# EUROPÄISCHE PATENTSCHRIFT

(11) **EP 0 746 262 B1**
(45) Veröffentlichungstag und Bekanntmachung des Hinweises auf die Patenterteilung: **21.08.2002**
(21) Anmeldenummer: 95909787.4
(22) Anmeldetag: 27.02.1995
(51) Int. Cl.: A61C 1/07, A61C 5/02, A61B 17/32, B06B 3/00, G10K 11/02

(54) **VORRICHTUNG ZUR ULTRASCHALLPRÄPARATION VON MENSCHLICHEN ODER TIERISCHEN HART- ODER WEICHGEWEBEN UND VON ZAHN- ODER KNOCHENERSATZMATERIALIEN**
DEVICE FOR THE ULTRASONIC PREPARATION OF HUMAN OR ANIMAL HARD OR SOFT TISSUES AND TOOTH OR BONE REPLACEMENT MATERIALS
DISPOSITIF DE PREPARATION AUX ULTRASONS DE TISSUS DURS OU MOUS D'ANIMAUX OU D'ETRES HUMAINS, AINSI QUE DE MATERIAUX DE REMPLACEMENT DE DENTS OU D'OS

(30) Priorität: 27.02.1994 DE 4406323
(43) Veröffentlichungstag der Anmeldung: 11.12.1996
(73) Patentinhaber: Hahn, Rainer, Dr., 72074 Tübingen (DE)
(72) Erfinder: Hahn, Rainer, Dr., 72074 Tübingen (DE)
(74) Vertreter: Ostertag, Reinhard
(86) Internationale Anmeldenummer: EP9500710
(87) Internationale Veröffentlichungsnummer: WO95022938

(56) Entgegenhaltungen:
- EP-A- 0 238 772
- EP-A- 0 594 541
- DE-A- 4 209 191
- DE-B- 1 100 424
- GB-A- 2 104 389
- US-A- 5 289 436

## Beschreibung

Die Erfindung betrifft eine Vorrichtung zur Ultraschallpräparation von menschlichem oder tierischem Hart- oder Weichgewebe sowie von Zahn- oder Knochenersatzmaterialien.

In der EP 0 594 541 A2, welche einen Stand der Technik gamäß Art. 54(3)(4) bildet, ist ein Ultraschall-Bearbeitungsgerät beschrieben, welches sich besonders zur Bearbeitung schwer zugänglicher Werkstücke eignet. Hierzu ist ein Ultraschallgenerator über einen Umlenkkopf mit einem Werkzeug gekoppelt, welcher als ringförmiger Biegeschwinger mit einer Mehrzahl in Umfangsrichtung verteilter Schwingungsknoten und Schwingungsbäuche ausgebildet ist. Das Abtriebsteil des Ultraschallgenerators und das getriebene Teil des Werkzeuges sind mit in Umfangsrichtung versetzten Punkten des Biegeschwingers verbunden.

Die subtraktive Bearbeitung natürlicher Hartgewebe, wie z.B. Zahnschmelz, Zahnbein, Zahnzement und Knochen sowie von Zahn- bzw. Knochenersatzmaterialien ist Grundlage nahezu jedes zahnärztlichen bzw. chirurgischen Eingriffes. Bis heute erfolgt die Bearbeitung der genannten Hartgewebe im Alltag mit rotierenden Diamant- oder Hartmetallwerkzeugen bzw. unter Verwendung von scharfen, z.B. meißelförmigen Handinstrumenten.

Derartige Bearbeitungswerkzeuge führen zu ausgeprägten Vibrationen, unangenehmen Geräuschbildungen und induzieren zum Teil erhebliche Schmerzen. Es besteht die Gefahr der Überhitzung der bearbeiteten vitalen Gewebe mit der Folge einer möglichen, irreversiblen Schädigung der organischen Bestandteile bzw. benachbarter Organe, wie z.B. der Zahnpulpa. Darüber hinaus ist ein selektiver Materialabtrag, z.B. kariös veränderter Zahnhartsubstanzen, unter Schonung angrenzenden gesunden Gewebes nicht möglich. Beim Schneideneingriff des Instrumentes kommt es nicht selten zu Ausprengungen und Rißbildungen innerhalb der spröden, anorganischen Gewebsbestandeile bzw. Gewebe, beispielsweise bis zum möglichen Abplatzen des Zahnschmelzmantels vom Zahnbein während der Schmelzbearbeitung mit rotierenden Diamantwerkzeugen. Darüber hinaus behindert das für eine anwendungsspezifische Mindeststeifigkeit erforderliche minimale Querschnitt-Längen-Verhältnis der Werkzeuge deren Handhabung in schwer zugänglichen Bereichen, wie z.B. in dünnen, langgestreckten Kavitäten, endodontischen Hohlräumen und Oberflächenabschnitten längs der Zahnwurzeln und zwischen diesen. Nicht zuletzt besteht eine hohe Verletzungsgefahr benachbarter Weichteile durch rotierende, schneidende Werkzeuge oder durch ein versehentliches Abgleiten, z.B. von Handwerkzeugen.

Die erzeugte Kavitätengeometrie ist das Ergebnis der geometrischen Form des z.B. rotierenden Werkzeuges sowie einer Relativbewegung zwischen Werkzeug und Werkstück; die Präparation konfektionierter geometrischer Formen ist nicht möglich.

Die indikationsspezifische Kavitätenpräparation erfolgt nach vorgegebenen Grundregeln (Expertenwissen), die jedoch aufgrund der individuell erzeugten Kavitätenformen nicht als "Informationen" zur Herstellung von z.B. Zahn- bzw. Knochenersatzteilen genutzt werden können. Die Anfertigung von Rekonstruktionen erfordert daher eine präzise Abformung sämtlicher bearbeiteten Oberflächen; kleinere Abbildungsfehler können nicht durch Verlaufsintegration der benachbarten, präzise abgeformten Oberflächenabschnitte, unter Berücksichtigung des Expertenwissens, korrigiert werden.

Die Präparation vitaler Hartgewebe mittels Laserenergie führt zu thermischen sowie thermoschockinduzierten mechanischen Schäden der bearbeiteten Hartsubstanzen bzw. benachbarter Gewebe und ist im Vergleich zu konventionellen Bearbeitungsverfahren aufwendig und unwirtschaftlich. Darüber hinaus ist die Präparation von definierten Kavitäten durch den unkontrollierbaren Tiefenabtrag sowie die nicht taktile, freie Handhabung der Werkzeuge erschwert. Ferner besteht die Gefahr der Schädigung der Weichgewebe durch direkte Bestrahlung bzw. Reflexionseffekte.

Die Präparation von Zahnhartgeweben mittels feiner Aluminiumoxidkorn-Strahlanlagen ist aufgrund des Abtragverfahrens, der kaum kontrollierbaren Handhabung und der zuvor notwendigen Applikation von Spanngummi auf ein schmales Anwendungsspektrum beschränkt und aufwendig. Darüber hinaus ist die Gefahr der Lungenschädigung des Patienten und des Behandlungspersonales durch unvermeidbare Staubbildungen hoch.

Die Präparation von Zahn- und Knochengeweben unter Anwendung von oszillierenden Werkzeugen wurde bereits vor Jahrzehnten beschrieben.

Die US 2,874,470 (High frequency dental tool, 1959) offenbart die Präparation von Zahnhartgeweben unter Anwendung magnetostriktiver, oszillierender Werkzeuge und abrasiver Füssigkeiten.

Die DE 11 00 424 beschreibt einen in der Schwingungsknotenebene geteilten Amplitudentransformator einer Ultraschallbohrvorrichtung zur Zahnbehandlung und erleichtert somit den Einsatz verschiedener Bearbeitungswerkzeuge.

Elektromechanische und magnetostriktive (Lamellen-)Transducer sind in der US 3,075,288 zum Zwecke der Zahnbehandlung mit oszillierenden Werkzeugen unter Einsatz abrasiver Flüssigkeiten offenbart.

Die daraus hervorgegangenen, modernen Ultraschallbearbeitungswerkzeuge bestehen z.B. aus einem piezoelektrischen Schwingungserreger mit an beiden Stirnflächen des Piezoquarzes koaxial befestigten, rotationssymetrischen Endmassen zur Schwingungsverstärkung durch Abstimmung des Gesamtsystemes in Resonanzfrequenz. Zur Amplitudenverstärkung haben sich ferner einseitig koaxial angebrachte rotationsymetrische Endmassen (Sonotroden) bewährt, die ebenfalls in Resonanzoszillation mit dem schwingungserregenden System abgestimmt sind und deren Querschnitt sich mit zunehmendem Abstand vom Piezoquarz bevorzugt verjüngt. Koaxial an die Stirnfläche der Sonotrode kann eine weitere, in Resonanzfrequenz abgestimmte Endmasse im Sinne eines Bearbeitungswerkzeuges angegliedert werden.

Das Gesamtsytem führt harmonische Longitudinalschwingungen längs seiner Längsachse aus, wobei die größten Amplituden an den dem Piezoquarz gegenüberliegenden Stirnflächen der Schwingungsverstärker vorliegen und das System wenigstens eine Knotenebene senkrecht zur Systemlängsachse (= Längsachse des Schwingungserregers) durch die Mitte des Piezoquarzes aufweist.

Der Betrieb der o.g. Zahnbearbeitungswerkzeuge im Ultraschallfrequenzbereich (etwa 18kHz bis 30kHz) resultiert aufgrund der physikalischen Beziehungen zwischen der Resonanzfrequenz und der Wellenlänge in ausreichenden Arbeitsamplituden des flüssigkeitsumspülten bevorzugt metallischen Werkzeuges und ermöglicht dadurch abtragswirksame Kavitationseffekte, insbesondere zur Zahnsteinentfernung. Andererseits bestimmt die Wellenlänge des in Resonanz betriebenen Ultraschallbearbeitungsgerätes dessen Baulänge in der Systemlängsachse und behindert daher die Anwendung in schwer zugänglichen Bereichen. Insbesondere ist eine Bearbeitung von Seitenzähnen durch die physiologisch limitierte Mundöffnung von etwa 40 bis 50 mm mit herkömmlichen Ultraschallbearbeitungswerkzeuges kaum realisierbar.

Im Bestreben, eine Lösung dieser Problematik zu finden, hat die Fa. Cavitron Ultrasonics im Jahre 1956 (US 492 924) Werkzeuge zur Ultraschallbearbeitung von Zahnhartsubstanzen beschrieben, die durch eine im Bezug zur Systemlängsachse exzentrische Massenverteilung gekennzeichnet sind. Derartige Werkzeuge sind auch durch die DE 1 258 017, die US 2, 990, 616 und einen wissenschaftlichen Artikel von H. H. Postle im J. Prosth. Dent. Vol.8, 1958, S. 153-160 offenbart. Die Schwingungsanregung der massenexzentrischen Werkzeuge in Systemlängsachse führt zur Induktion von transversalen, insbesondere ellipsoiden Raumschwingungen des Bearbeitungswerkzeuges, sodaß in jeder Raumrichtung ein Amplitudenanteil vorliegt. Dieser Amplitudenanteil ist allerdings entlang der meist senkrecht zur Systemlängsachse (Handstückachse) verlaufenden Präparationsrichtung zur Erzeugung von Zahnkavitäten nicht ausreichend. Die Raumschwingungen verhindern ferner die Ausbildung eines kontinuierlichen Flüssigkeitsfilmes zwischen dem Werkzeug und der zu bearbeitenden Oberfläche, die jedoch Voraussetzung für eine Energieübertragung und einen Materialabtrag, z.B. durch Kavitationseffekte, ist.

Ferner kommt es durch die in den verschiedenen Raumrichtungen unkontrollierbaren, teilweise hohen Amplituden zur mechanischen Schädigung der z.T. spröden Zahnhartgewebe (H. Sprange und G. Haim, ZWR Vol. 22, 1969 S. 1028-1031). Darüber hinaus resultiert die Entfernung von Zahnbelägen mittels derartiger Ultraschallwerkzeuge, die im wesentlichen noch heute unverändert in Gebrauch sind, in einer signifikanten Aufrauhung der bearbeiteten Zahnoberflächen, was die Neubildung von Belägen begünstigt. Nicht zuletzt birgt die hohe Amplitude die Gefahr thermischer Schädigungen von Hartund Weichgeweben.

Der Einsatz von z.B. zahnärztlichen Ultraschallwerkzeugen unter kontinuierlicher Wasserkühlung ist daher auf die Entfernung von supragingivalem Zahnstein beschränkt, eine Anwendung zur Präparation von definierten Kavitäten, Reinigung von subgingivalen Zahnoberflächen insbesondere in Zahnfleischtaschen, einer wünschenswerten spanbildenden Aufbereitung endodontischer Hohlräume sowie eine Bearbeitung von Zahn- bzw. Knochenersatzwerkstoffen ist bislang nicht möglich.

Ultraschallangetriebene rotierende Werkzeuge, die herkömmliche schneidende Bearbeitungswerkzeuge einsetzen (Diamantwerkzeuge mit gebundenem Korn oder schneidende Hartmetallwerkzeuge) sind in der US 4,281,987, der US 4,289,849 sowie durch ein Übersichtsreferat von L. Balamuth, IEEE, 1963 S. 96-101 offenbart. Die Abtragsmechanismen unterscheiden sich nicht von herkömmlichen, rotierenden (luftoder motorgetriebenen) Werkzeugen, wobei der Ultraschallantrieb vergleichsweise unwirtschaftlich ist.

Die AT 290 005 beschreibt eine Vorrichtung zur reproduzierbaren Abtragung von Zähnen mit Hilfe einer "stationären" Hülse, die scheinbar unter dem Einfluß eines "Ultraschallfeldes" steht. Das "Ultraschallfeld" ist nicht näher beschrieben, obgleich die beigefügte Graphik die Handhabung der Hülse im Seitenzahnbereich darstellt.

Mit den bis zum heutigen Tage bekannten Ultraschallbearbeitungsgeräten ist es aufgrund o.g. physikalischer Grundlagen, insbesondere im Seitenzahnbereich, nicht möglich eine solche Hülse mit parallel zur Zahnlängsachse im Sinne einer Materialzerspanung wirksamen longitudinalen Arbeitsamplituden zu versehen. Lateral- bzw. Raumschwingungen würden in dieser Anwendung zu einer Überlagerung des Schwingungsbildes mit der konfektionierten Werkzeugform und dadurch zu massiven Abbildungsfehlern beim Einsenken der Hülse führen. Eine Rekonstruktion durch in Analogie zur Hülsenform vorgefertigte Konfektionskronen ist bei Raumschwingungen des Bearbeitungswerkzeuges bzw. der Hülse aufgrund der aus der Schwingungsgeometrie resultierenden Forminkongruenzen zwischen den bearbeiteten Flächen des Zahnstumpfes und den Innenflächen der Restauration nicht möglich.

Andererseits ist der Einsatz von Konfektionswerkzeugen, wie der o.g. Hülse oder das in der US 2,874,470 offenbarte Werkzeug zur Präparation einer gesamten Inlaykavität aufgrund der individuellen Zahnformen und der variablen Zahngrößen wenig substanzschonend und bietet keine Sicherheit z.B. bzgl. einer vollständigen Entfernung erkrankter Gewebeabschnitte. Nicht zuletzt besteht die Gefahr einer unbeabsichtigten Eröffnung benachbarter, z.B. endodontischer Hohlräume.

Die Aufgabe der vorliegenden Erfindung ist es daher, eine Ultraschallbearbeitungs-Vorrichtung anzugeben, bei der abtragswirksame Arbeitsamplituden am Werkzeug erhalten werden, die eine wesentlich kürzere Baulänge als die bisherigen Ultraschallbearbeitungsgeräte aufweist und wie ein herkömmliches Winkel-Handstück ergonomisch günstig gehandhabt werden kann und dadurch die wirtschaftliche Verwendung eines solchen Werkzeuges in schwer zugänglichen Bereichen, z.B. bei der intraoralen Hartgewebsbearbeitung, ermöglicht.

Diese Aufgabe ist durch eine Vorrichtung gemäß Anspruch 1 gelöst.

Diese hat mindestens einen, zwischen einem Schwingungsgenerator und einem Bearbeitungswerkzeug angebrachten Umlenkkopf, der parallel zur Längsachse des Schwingungsgenerators zur Oszillation angeregt wird und eine Abtriebsbewegung in Längsrichtung des Bearbeitungswerkzeuges bereitstellt, wobei die Längsachse des Schwingungsgenerators mit der des Bearbeitungswerkzeuges einen Winkel von 60° bis 120° bildet.

Derartige Umlenkköpfe können sogar in wesentlich geringerer Bauhöhe gefertigt werden, als das schwingungserzeugende System selbst, was das Handhaben des Bearbeitungswerkzeuges weiter erleichtert.

Die geometrische Form des die Richtung der Ultraschalloszillation ändernden Umlenkkopfes ist anwendungsspezifisch ausgebildet, und handelt es sich bei ihm um einen Schwingkörper mit mehreren Schwingungsbäuchen, die das getriebene und treibende Teil des Umlenkkopfes bilden, so bestimmt sie zusammen mit dem(n) verwendeten Material(ien) seine Eigenfrequenz sowie die in Richtung des Bearbeitungswerkzeuges induzierte Schwingungsform.

Besonders geeignet zur Herstellung von Festkörper-Umlenkkopf-Schwingkörpern sind elastische, bevorzugt metallische Werkstoffe, insbesondere C-Stähle mit bevorzugt martensitischem oder bainitischem Gefüge, die besonders bevorzugt oberflächenvergütet und/oder konditioniert werden, oder Titan- oder Bronzelegierungen.

Die geometrische Form von Festkörper-Schwingkörpern sollte eine elastische Oszillation derselben in Resonanz mit dem schwingungserregenden System ermöglichen, wobei sich der Schwingkörper sowohl in Längsrichtung des schwingungserregenden Systemes bevorzugt ohne Phasenverschiebung verformt, als auch in Achsrichtungen von 60° bis 120° zur Längsachse des schwingungserregenden Systems oszilliert. Dies bedeutet unter Berücksichtigung des Winkelversatzes der Koordinatenrichtungen oder bei Einführung einer gebrochenen Koordinate, die vom Schwingungsgenerator über den Umlenkkopf durch das Werkzeug läuft, eine gleichphasige Bewegung von Schwingungsgenerator-Abtriebsteil und Werkzeug.

Das Bearbeitungswerkzeug ist am Umlenkkopf in einem Winkel von 60° bis 120°, besonders bevorzugt von 90° zur Längsachse des schwingungserregenden Systems angebracht und bzgl. seiner Form, Länge und Masse so ausgebildet, daß es in Resonanz mit dem Umlenkkopf bzw. dem schwingungserregenden System oszilliert.

Besonders geeignet zur intraoralen Anwendung sind kugelförmige, scheibenförmige oder ringförmige Umlenk-Schwingkörper mit einem Durchmesser von weniger als 30 mm, insbesondere weniger als 20 mm. Zur Verbesserung des Wirkungsgrades hat es sich ferner bewährt, hohlkugelförmige, ringförmige oder insbesondere zylinderhülsenförmige Schwingkörper zu verwenden.

Zur Verbindung des Umlenkkopfs mit dem schwingungserregenden System sowie des Bearbeitungswerkzeugs mit dem Umlenkkopf eigenen sich herkömmliche Fügetechniken sowie teilbare Verbindungen. Insbesondere kann der Umlenkkopf mit dem schwingungserregenden System sowie das Bearbeitungswerkzeug mit dem Umlenkkopf verlötet, verschweißt, verklebt, verschraubt, verspannt, verkeilt, oder mit Hilfe eines Friktionskonus verbunden sein, wobei sich insbesondere die Kombination einer Schraubverbindung mit einem Friktionskonus bewährt hat. Selbstverständlich kann der Schwingungsverstärker des schwingungserregenden Systems und der Umlenkkopf und/oder das Bearbeitungswerkzeug und der Umlenkkopf auch aus einem Stück gefertigt werden. Generell erfolgt die Herstellung der Verbindungen in einer durch die Achse des Schwingungsgenerators und die Werkzeugachse vorgegebenen Symmetrieebene des Umlenkkopfes.

Als Bearbeitungswerkzeuge eignen sich insbesondere metallische Werkzeuge, wie z.B. zylinderförmige, röhrchenförmige, flammenförmige, kugelförmige, knospenförmige oder kegelförmige Werkzeuge, wie sie z.B. auch in der Zahnheilkunde gebräuchlich sind, jedoch bevorzugt ohne oberflächengebundenes Korn. Eine Kavitätenpräparation erfolgt unter wenigstens teilweiser Einsenkung des Werkzeuges und/oder durch Relativbewegung zwischen dem Werkzeug und der zu bearbeitenden Oberfläche. Ferner können definierte Formwerkzeuge verwendet werden, die wenigstens teilweise parallel zur Formwerkzeuglängsachse in die zu bearbeitende Oberfläche eingesenkt werden.

Die Zahnreinigung erfolgt mit Werkzeugen, die ähnliche Form wie ein Golf- oder Hockeyschläger haben können oder flächige schaufelähnliche Arbeitsteile haben und gemäß der Zahnkrümmung gewölbt sind.

Zur Energieankopplung an die zu bearbeitenden Oberflächen wird das oszillierende Werkzeug kontinuierlich mit einem flüssigen oder thixotrop gelförmigen Behandlungsmedium umspült, z.B. Wasser oder wässrigen Lösungen chemischer Wirksubstanzen, sodaß zwischen dem Werkzeug und der bearbeiteten Oberfläche eine geschlossene Flüssigkeitsschicht liegt.

Die schnelle Auf- und Abwärtsbewegung des oszillierenden Bearbeitungswerkzeuges führt in der unmittelbaren Umgebung des Werkzeuges, insbesondere der Werkzeugstirnseite zu Kavitationseffekten sowie zur Implosion von Kavitationsblasen im flüssigkeitsgefüllten Arbeitsspalt, die in einer spanabhebenden Oberflächenumformung nichtmetallischer Materialien, im Sinne eines Erosionsprozesses resultieren. Andererseits ermöglicht die oszillierende mikrospanabhebende Bearbeitung der Zahnhartsubstanzen eine weitgehend schmerzfreie Behandlung unter Vermeidung einer Anästhesie.

Der Spanabtrag wird mit zunehmendem Arbeitsabstand geringer und kommt mit Abreißen des Flüssigkeitsfilmes zum Stillstand. Ein zu geringer Arbeitsabstand bei zu hohem Anpressdruck des oszillierenden Bearbeitungswerkzeuges an die zu bearbeitenden Oberflächen wirkt der Ausbildung von Kavitationseffekten entgegen und führt zum Prozeßstillstand.

Andererseits kann durch unterschiedliche manuelle Anpressdrucke des Bearbeitungswerkzeuges gegen die zu bearbeitende Oberfläche der Arbeitsabstand und damit die Energieankopplung variiert werden, was erstmalig die Möglichkeit eröffnet, unter Verzicht auf einen Werkzeugwechsel bzw. ohne Änderung am Bearbeitungsgerät, behandelte Oberflächen abtragend zu bearbeiten und schonend zu finieren bzw. zu polieren.

Darüber hinaus hat es sich bewährt dem Behandlungsmedium zur Steigerung der Abtragsleistung feine abrasive Hartkörner (nachstehend kurz: Abrasivpartikel), wie z.B. Metalloxidpartikel, insbesondere Aluminiumoxidpartikel, Magnesiumoxidpartikel, Siliziumnitridpartikel, Borkarbidpartikel oder Glaspartikel, bzw. feine Diamantkörnchen beizumengen, wobei die Korngröße der Partikel, insbesondere für eine optimale Beschleunigung im Arbeitsspalt und eine daraus resultierende optimale Abtragsleistung, etwa in der Größenordnung der doppelten Amplitude, also dem Gesamthub der Ultraschallschwingunges Bearbeitungswerkzeuges liegen sollten. Es hat sich bewährt Abrasivsuspensionen einer Zusammensetzung von einem Volumenanteil Abrasivpartikel auf 3 bis 50, bevorzugt 5 bis 20 und ganz besonders bevorzugt von 10 Volumenanteilen Flüssigkeit zu verwenden, wobei die Körner, z.B. durch kontinuierliches Rühren oder durch Gasdurchflutung der Suspension, z.B. in einem auswechselbaren Vorratsbehälter, in Schwebe gehalten werden.

Wie überraschend gefunden wurde, können die bei der Präparation von Zahnbein eröffneten Dentinkanälchen durch zusätzliches Beimengen von Feinkornteilchen (nachstehend auch: Versiegelungspartikel) in die Suspension während des Abtragsprozesses, im Sinne eines Wundverbandes, versiegelt werden. Insbesondere hat sich die Zugabe von scharfkantigen unrunden (nachstehend kurz: blockförmigen) Metalloxidfeinpartikeln, insbesondere Aluminiumoxidfeinpartikeln mit einem mittleren Durchmesser von weniger als 3 µm, insbesondere von etwa 1 µm bewährt. Bewährte Mischungsverhältnisse liegen bei einem Volumenanteil Feinkornteilchen auf 2 bis 20, bevorzugt etwa 10 Volumenanteile Abrasivpartikel.

Die erfindungsgemäße Versiegelung der während der Präparation angeschnittenen Dentinkanälchen mit Feinkornteilchen führt zu einer signifikanten Reduktion der Dentinpermeabilität und erübrigt einen aufwendigen Wundverband bzw. eine Unterfüllung.

Andererseits steht durch die zugfeste Verkeilung der insbesondere blockförmigen Feinkornteilchen in der bearbeiteten Dentingrenzfläche erstmals ein definiertes Substrat zur Verfügung, das unter Umgehung einer problematischen Applikation von Dentinätzlösungen und/oder Dentinhaftvermittlern mit geringfügiger Haftungsverbesserung, zur Haftvermittlung von plastischen Zahnfüllmaterialien, insbesondere Polymerkomposits oder Glasionomerzementen, verwendet werden kann.

Überraschenderweise können wenigstens teilweise silikathaltige und silanisierte sowie wenigstens teilweise polymerhaltige Granulate einen direkten chemischen Verbund mit polymerisierbaren Zahnfüllmassen eingehen, wobei eine mögliche Verschiebung der Granulate in den Dentingrenzflächen der Polymerisationsschwindung der Zahnfüllmaterialien entgegenwirkt. Ferner können wenigstens teilweise silikathaltige Granulate z.B. mit polyacrylsäurehaltigen Füllungszementen gelieren.

Die Zuleitung der Abrasivpartikel-Feinkornteilchen-Wasser-Suspension (Slurry) erfolgt z.B. über Düsen am Handstück oder Bearbeitungswerkzeug, insbesondere über eine zirkulär um das Bearbeitungswerkzeug angeordnete Ringdüse oder durch etwa röhrenförmige Bearbeitungswerkzeuge. Andererseits kann bei äußerer Slurryzuleitung die Abtragseffizienz sowie die Sichtkontrolle, insbesondere bei tiefen Kavitäten, durch eine durch röhrenförmige Bearbeitungswerkzeuge erfolgende Absaugung verbessert werden.

Alternativ bzw. in Ergänzung zu wässrigen Suspensionen können des weiteren etwa gelartige Kornaufschwemmungen der o.g. Abrasivpartikel und/oder Feinkornteilchen, beispielsweise in Glyceringel oder Gelatine oder 1-10%-igem Chlorhexidindigluconat-Gel, verwendet werden. Die Viskosität derartiger Gele kann z.B. durch Zugabe von Aerosilen anwendungsspezifisch variiert werden und resultiert in einer möglichen selektiven Applikation, insbesondere auch entgegen der Schwerkraft sowie in einem möglichem Verzicht auf eine kontinuierliche wässrige Schleifmittelzuführung bzw. Absaugung. Durch Ultraschalleinwirkung kommt es durch die Thixotropie des Geles zu einer selektiven Viskositätsverringerung in der unmittelbaren Umgebung des oszillierenden Werkzeuges und dadurch zu effizienten Kavitationseffekten bzw. einer ausreichenden Schleifmittelbeschleunigung im Arbeitsspalt.

Besonders gut hat sich der Einsatz derartiger Gele in schwer zugänglichen Bereichen, wie z.B. in Zahnfleischtaschen, Zahnzwischenräumen oder in endodontischen Hohlräumen sowie zur Ausarbeitung von Zahnrestaurationen, vorzugsweise als Alternative zu einer kontinuierlichen Schleifmittelzuführung bewährt .

Selbstverständlich können den Flüssigkeiten bzw. Gelen auch chemische Wirksubstanzen zugesetzt werden, wie z.B. Calziumchelatbildner (EDTA-Lösung) zur chemischen Unterstützung des Hartgewebeabtrages oder Natriumhypochloritlösung zur Auflösung von Weichgeweberesten, z.B. im Zuge der Aufbereitung endodontischer Hohlräume oder organische Säurelösungen zur gleichzeitigen Entfernung bearbeitungsinduzierter Schmierschichten und/oder zur mikromorphologischen Umstrukturierung der bearbeiteten Oberflächen oder Wirksubstanzen zur Reduktion der Keimzahlen an den behandelten Oberflächen bzw. in deren Umgebung (z. B. Keimreduktion in Zahnfleischtaschen).

So hat es sich z.B. zum selektiven Abtrag von kariösen Zahnhartsubstanzen bewährt auf die Zugabe von groben Abrasivpartikeln zu verzichten und statt dessen Flüssigkeiten bzw. Gele unter Zugabe von etwa Natriumhypochloridlösung zu verwenden.

Selbstverständlich können auch hochvisköse Flüssigkeiten, z.B. Polymerkomposits unter Ultraschalleinwirkung thixotrop verflüssigt und z.B. als Befestigungskomposits zwischen Zahnkavitäten und mit Ultraschall beaufschlagten Zahnrestaurationen zu einem dünnen Film ausgepresst werden.

Die in der Zahnheilkunde durch spanabhebende Entfernung kariöser Zahnhartsubstanzen resultierenden Hartgewebsdefekte werden zur Verbesserung etwa der Stabilität oder der Randständigkeit der einzugliedernden Restaurationen unter Beachtung exakter Präparationsrichtlinien (Expertenwissen), im Sinne von Kavitäten bzw. Zahnstümpfen präpariert.

Intraorale Kavitäten, z.B. zur Aufnahme von Einlagefüllungen oder Teilkronen, können jeweils in ein oder mehrere, im Sinne des Expertenwissens definierbare, okklusale Kavitätensegmente und/oder ein oder mehrere approximale bzw. bukkale bzw. orale Kavitätensegmente mit jeweils divergierenden gegenüberliegenden Wänden unterteilt werden.

Erfindungsgemäß wird dieses Expertenwissen in ein Set standardisierter Bearbeitungsformwerkzeuge in verschiedenen Größenabstufungen umgesetzt, wobei jedes Bearbeitungswerkzeug als Negativform des zu präparierenden Kavitätensegmentes ausgebildet ist. Insbesondere hat sich die Herstellung wenigstens eines Formwerkzeuges zur Bearbeitung okklusaler Kavitätensegmente und/oder wenigstens eines Formwerkzeuges zur Bearbeitung approximaler, bukkaler oder oraler Kavitätensegmente jeweils in wenigstens einer Größe, vorzugsweise in verschiedenen Größenabstufungen bewährt. Die Kavitätenpräparation schließt sich der individuellen Entfernung z.B. kariöser Zahnhartsubstanz an und erfolgt durch längsachsenparalleles Einsenken ein oder mehrerer erfindungsgemäßer, oszillierender Formwerkzeuge in die bevorzugt vorbearbeiteten Oberflächen. Zusammengesetzte Kavitätenformen resultieren aus dem benachbarten Einsenken verschiedenener Segmentwerkzeuge, insbesondere nach Vorauswahl in geeigneten Formwerkzeuggrößen.

Die erfindungsgemäße Restauration derartiger Kavitäten erfolgt durch Eingliederung wenigstens eines standardisierten, mit dem(n) verwendeten Bearbeitungsinstrumen(en) formgleichen Paß- oder Füllkörpern, der(die) aus einem bezüglich der Formen und Größenabstufungen mit den korrespondiereneden Formwerkzeuggeometrien übereinstimmenden Set an Formkörpern ausgewählt wird(werden). Insbesondere hat sich die Anwendung adhäsiver Restaurationsverfahren unter Verwendung von Polymerkomposits sowie eine materialspezifische Oberflächenkonditionierung der Kavitätenoberflächen und der den Kavitätenoberflächen bzw. benachbarter Füllkörpersegmente zugewandten Füllkörperoberflächen bewährt.

Die Kavitätenpräparation und definitive Restauration erfolgt somit in der selben Behandlungssitzung unter Umgehung komplizierter Abformtechniken sowie einer aufwendigen Laborfertigung individueller Paßkörper.

Darüber hinaus können erfindungsgemäße Formwerkzeuge auch zur Präparation von Oberflächensegmenten insbesondere von Zahnstumpfoberflächen, z.B. im Rahmen der Präparation von Verblendschalen oder von Kronenstümpfen, verwendet werden. Wiederum findet das Expertenwissen bezüglich der Gestaltung der etwa parallel zur Zahnlängsachse verlaufenden Stumpfoberflächen Eingang in ein Set etwa schaufelförmiger Präparationswerkzeuge in unterschiedlichen Größenabstufungen bzw. unterschiedlichern Krümmungsradien. Insbesondere kann das Expertenwissen zur Präparation von Randübergängen, bevorzugt von Kronenrändern, z.B. in Form von Stufen- oder Hohlkehlübergängen jeweils mit oder ohne Randanschrägung in ein Bearbeitungsformwerkzeug, das wenigstens teilweise als Negativ der zu bearbeitenden Oberflächenformen ausgebildet ist, überführt werden. Die Präparation von Randübergängen erfolgt vorzugsweise durch zirkuläres Führen derartiger Werkzeuge parallel zur Formwerkzeuglängsachse um den gesamten umzugestaltenden bzw. zu finierenden Randübergang. Ganz besonders bewährt hat sich diese Methode, da eventuelle Abformungenauigkeiten durch Verlaufsintegration benachbarter, präzise abgeformter Modellstumpfoberflächenabschnitte unter Berücksichtigung des Expertenwissens, bevorzugt durch Nachbearbeitung der Modellstümpfe mit identischen Bearbeitungsformwerkzeugen, verlaufsintegriert werden können.

Die Verwendung eines Sets von in Analogie zu Zahnwurzeloberflächen bzw. -oberflächensegmenten ausgebildeten, etwa schaufelförmigen oder die Form von Golf- oder Hockeyschlägern aufweisenden Werkzeugen erlaubt ferner die Entfernung von Zahnbelägen und adsorbierten Hartstoffen, etwa Zahnstein, von supra- und subgingivalen Wurzeloberflächen, wobei die Zusammensetzung des verwendeten Arbeitsmediums (z.B. Wasser oder abrasive Suspension) sowie die Schwingungsform der Werkzeuge eine weitgehend selektive Entfernung des Zahnsteines und ggf. von Konkrementen ermöglicht, ohne daß größere Volumina gesunden Zahnhartgewebes abgetragen oder z.B. im Sinne einer Oberflächenaufrauhung verändert werden.

Darüber hinaus können durch Verwendung von wenigstens teilweise ovalen Bearbeitungswerkzeugen erstmals endodontische Hohläume intrumentell entsprechend ihrer in Analogie zum Wurzelquerschnitt zumeist ovalen Form aufbereitet werden.

Überraschenderweise wurde ferner entdeckt, daß etwa drahtförmige, zylinderförmige oder schneidenartig ausgebildete Bearbeitungswerkzeuge aus etwa Chrom-Nickelhaltigen Metallegierungen unter Ultraschalleinwirkung ohne Wasserkühlung durch innere Reibungseffekte selektiv erhitzt und zur schneidenden Bearbeitung von Weichgeweben bzw. zur Koagulation von eröffneten Blutgefäßen eingesetzt werden können. Besonders bewährt haben sich Bearbeitungswerkzeuge, die die Ausbildung von Reibungswärme unter Ultraschalleinwirkung z.B. durch Zusammensetzung aus wenigstens zwei verschiedenen Materialien bzw. durch Induktion von Relativbewegungen des Bearbeitungswerkzeuges zum Umlenkkopf begünstigen. Mit solchen Werkzeugen können Weichteilgewebsschnitte in Analogie zu CO₂ -Lasern bzw. zu elektrischen Hochfrequenzwerkzeugen ausgeführt werden, ohne daß es aufgrund von unerwünschten optischen Reflexionsphänomenen oder elektrischen Funkenbildungen zu Verletzungen kommt.

Wärmeerzeugende Werkzeuge können insbesondere auch in der Endodontie nach erfolgter Wurzelkanalaufbereitung vorzugsweise unter Verwendung formgleicher Bearbeitungswerkzeuge als Füllkörper verwendet werden. Die Wärmeinduktion ermöglicht dabei die Verarbeitung von z.B. thermoplastischen Wurzelfüllmaterialien, die durch das oszillierende, wärmeinduzierende Werkzeug wenigstens teilweise verflüssigt und unter Vorschub des Werkzeuges in den Wurzelkanal zu einem geringen Volumen ausgepresst werden und nach Abkühlung durch Abschalten der Ultraschalloszillation, bevorzugt unter Adsorption auf die Zahnhartgewebs- und Bearbeitungswerkzeugoberflächen, als Fügematerial (Sealer) dienen, welches die indviduellen Hohlräume zwischen den Wurzelkanaldentinwänden und dem gleichzeitig als Makro-Füllkörper dienenden Bearbeitungswerkzeug verschließen. Die durch die konische Form Werkzeuges gerichtete Abkühlung von der Spitze zur Werkzeugbasis ermöglicht ein spaltfreies Versiegeln der Hohlräume zwischen dem Werkzeug und der Wurzelkanalwand, wobei das Werkzeug nach Ablösen vom Umlenkkopf vorzugsweise als geometrisch definierter Formkörper im Wurzelkanal, im Sinne eines Füllkörpers, belassen und bevorzugt auf die individuelle Länge der Zahnwurzel gekürzt wird.

Selbstverständlich ist die Verwendung der erfindungsgemäßen Präparationsvorrichtung nicht auf die Anwendung in der Zahnheilkunde beschränkt; diese ist auch in äquivalenten medizinischen und nichtmedizinischen und auch industriellen Aufgabenstellungen anwendbar.

Nachstehend wird die Erfindung anhand von Ausführungsbeispielen unter Bezugnahme auf die Zeichnung näher erläutert. In dieser zeigen:
- Figur 1:: einen axialen Schnitt durch das Ende eines zahnärztlichen Winkel-Handstückes zur Bearbeitung von Zahnmaterial mit einem Festkörper-Schall-Umlenkteil wiedergegeben;
- Figur 2:: eine getrennte Darstellung des Schall-Umlenkteiles von Figur 1, anhand dessen dessen Funktionsweise näher erläutert wird; und
- Figur 3:: eine seitliche Ansicht einer Kavitätempräparation mit einem Ultraschall-Formwerkzeug, welches zugleich als Form- oder Füllkörper dient;
- Figur 4:: einen ersten Schritt der Präparation eines ausgedehnten kariösen Bereiches eines Zahnes mit einem Handstück gemäß Figur 1, von der Seite her gesehen, in welchem ein zur Seite hin offener Zahnausschnitt erzeugt wird;
- Figur 5:: einen Schnitt längs der Linie V - V von Figur 4;
- Figur 6:: einen auf den Präparationsschritt von Figur 4 folgenden zweiten Präparationsschritt, in welchem eine mittige Ausnehmung im Zahn erzeugt wird, von der Seite her gesehen;
- Figur 7:: einen Schnitt längs der Schnittlinie VII - VII von Figur 6;
- Figur 8:: einen weiteren Schritt der Zahnpräparation und -restauration, der auf die Schritte nach den Figuren 4 bis 6 und einen nicht gezeigten weiteren Schritt folgt und in welchem in die zuvor erzeugte mittige Zahnausnehmung ein Füllkörper aus Zahnersatzmaterial eingesetzt wird, in seitlicher Richtung gesehen;
- Figur 9:: einen Schnitt längs der Linie IX - IX von Figur 8;
- Figur 10:: eine seitliche Ansicht eines Arbeitsschrittes, wie er zur Präparation eines Zahnstumpfes vor dem Anbringen einer Krone durchzuführen ist;
- Figur 11:: einen Schnitt längs der Linie XI - XI von Figur 10;
- Figur 12:: eine seitliche Ansicht eines Arbeitsschrittes zur Behandlung der oberen Endabschnitte der Außenfläche der Zahnwurzel mit einem Ultraschallwerkzeug;
- Figur 13:: weitere zahnärztliche Arbeiten unter Verwendung von Ultraschall-Werkzeugen, bei denen Fleisch geschnitten (linke Figurenhälfte) bzw. Knochenmaterial abgetragen (rechte Figurenhälfte) wird;
- Figur 14:: eine seitliche Ansicht eines Wurzelkanal-Behandlungsschrittes mit einem Ultraschall-Werkzeug; und
- Figur 15:: einen Schnitt längs der Linie XV - XV von Figur 14.

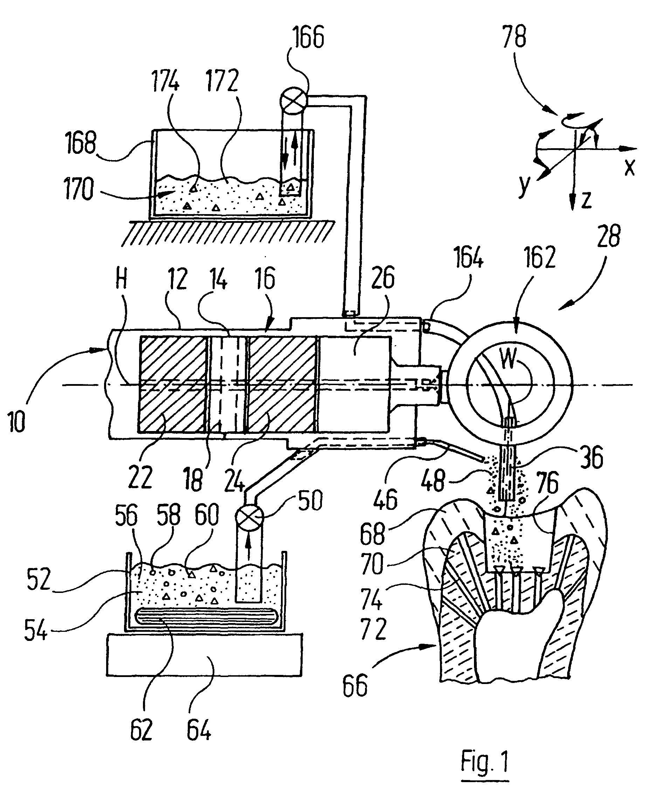

In Figur 1 ist das Ende eines insgesamt mit 10 bezeichneten zahnärztlichen Winkel-Handstückes wiedergegeben. Das Handstück 10 hat ein zugleich als Griff dienendes rohrförmiges Gehäuse 12, in welchem über Aufhängungen 14 ein insgesamt mit 16 bezeichneter Ultraschall-Schwingungsgenerator angebracht ist.

Der Ultraschall-Schwingungsgenerator 16 umfaßt einen piezoelektrischen Ultraschallwandler 18, der seinerseits aus mehreren axial hintereinanderliegenden und mechanisch in Reihe geschalteten Scheiben aus piezoelektrischem Keramikmaterial bestehen kann. Auf den Stirnflächen des Ultraschallwandlers 18 sind rotationssymmetrische Massenkörper 22, 24 angebracht, z.B. verschraubt oder angeklebt.

An dem in der Zeichnung rechts gelegenen Ende des Massenkörpers 24 ist ein Verstärkerkörper 26 angebracht, der die an seiner linken Stirnfläche erhaltene Ultraschallenergie auf Grund seiner zunehmenden Querschnittsabnahme zum freien Ende hin konzentriert und so für eine Amplitudenverstärkung sorgt. Derartige Verstärkerkörper werden oft auch als Sonotrode bezeichnet.

Das rechts gelegene Ende des Gehäuses 12 trägt einen insgesamt mit 28 bezeichneten Umlenkkopf, der ein Werkzeug 36 antreibt.

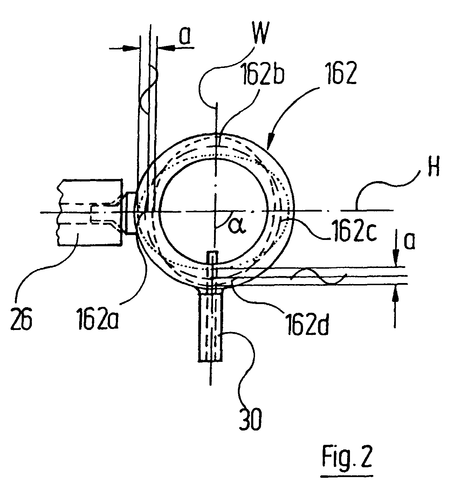

Der Umlenkkopf 28 weist einen Ringkörper 162 auf, der bezüglich seines Materiales und seiner Geometrie so gewählt ist, daß er vier um 90° in Umfangsrichtung versetzte Schwingungsbäuche aufweist, wie in Figur 2 angedeutet, die in Ringabschnitten 162a bis 162d liegen. An den einen Schwingungsbauch im Ringabschnitt 162a ist das Ende des Verstärkerkörpers 26 angekoppelt; der um 90° zur Achse des Schwingungsgenerators 16 nach unten versetzte Schwingungsbauch im Ringabschnitt 162d ist mit dem Werkzeug 36 verbunden. Der Ringkörper 162 besorgt somit eine Winkelumlenkung der Ultraschallenergie ohne Amplitudenverstärkung. Der Ringkörper 162 hat senkrecht zur Zeichenebene, also in axialer Richtung so große Abmessung, so daß seine Schwingungsmoden reine Atmungsbewegungen sind und keine oder nur in vernachlässigbarem Ausmaße Torsionskomponenten in Umfangsrichtung beinhalten.

Durch Auslegung des Ringkörpers 162 für drei Schwingungsbäuche kann man einen Winkel zwischen der Handstückachse H und der Werkzeugachse W (Umlenkwinkel) erhalten der 120° beträgt. Bei n Schwingungsbäuchen kann man Umlenkwinkel von 360°/n und ganzzahlige Vielfache hiervon realisieren.

Wünscht man Zwischenwinkel kann man solche durch Variieren der Dicke oder der axialen Erstreckung der Wand des Ringkörpers in Umfangsrichtung erhalten, da dann die Schwingungsknoten nicht mehr gleich in Umfangsrichtung verteilt sind.

In Abwandlung der oben betrachteten Ausführungsbeispiele für den Ringkörper kann man eine asymmetrische Ausbildung des Ringkörpers auch so wählen, daß man an dem die Abtriebsbewegung bereitstellenden Ringabschnitt eine höhere Amplitude erhält als am getriebenen Ringabschnitt. Der Ringkörper ist dann nicht mehr voll rotationssymmetrisch, hat z.B. in Umfangsrichtung variierende Wandstärke oder axiale Abmessung.

In weiterer Abwandlung kann man dann, wenn man nicht die volle Amplitude der vom Schwingungsgenerator 16 erzeugten Schwingung am Werkzeug benötigt, das Werkzeug 36 auch an einem Ringabschnitt des Ringkörpers 162 anbringen, der zwischen einem Schwingungsbauch und einem Schwingungsknoten liegt.

Figur 2 ist auch entnehmbar, daß dann, wenn durch den Schwingungsgenerator auf den Ringabschnitt 162a Druck ausgeübt wird, auch vom Ringabschnitt 162d auf das Werkzeug Druck ausgeübt wird. Die Kraftübertragung vom Schwingungsgenerator auf das Werkzeug erfolgt somit ohne Phasenänderung.

Am Gehäuseende ist ein Abgaberohr 46 für ein abrasives Behandlungsmedium 48 vorgesehen. Das Abgaberohr 46 ist mit dem Ausgang einer Pumpe 50 verbunden, welche das abrasive Behandlungsmedium aus einem Mischbehälter 52 ansaugt.

Alternativ kann man anstelle des Abgaberohres 46 oder zusätzlich zu diesem eine Ringdüse verwenden, die das Werkzeug 36 umgibt, vorzugsweise bei dessen oberen Ende, und über eine Mehrzahl zum vorderen Endabschnitt des Werkzeuges 36 gerichteter Düsen abrasives Behandlungsmedium abgibt.

Der im Mischbehälter 52 befindliche Mischungsvorrat 54 besteht aus Wasser, in welchem große Abrasivpartikel 56, mittlere Abrasivpartikel 58 und feine abrasive Versiegelungspartikel 60 enthalten sind. Diese sind schematisch durch Punkte, kleine Kreise und kleine Dreiecke unterschieden.

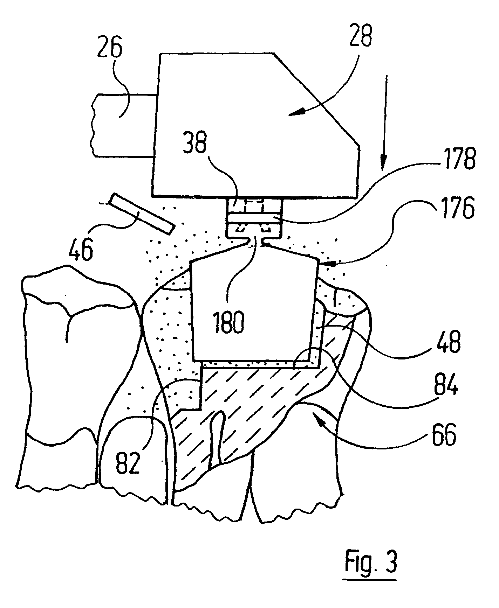

Die großen Abrasivpartikel 56 haben einen Durchmesser, der im wesentlichen dem Gesamthub der Ultraschall-Schwingungsbewegung des Werkzeuges 36 entspricht, also etwa a (vgl. Figur 3) beträgt. In der Praxis liegen somit die Durchmesser für die großen Abrasivpartikel unter 100 µm. Die großen Abrasivpartikel 56 haben eine kantenreiche durch Brechen oder Mahlen von Hartmaterial erhaltene Oberfläche und damit gute Schneideigenschaften. Das Material der großen abrasiven Hartkörner wird im Hinblick auf die jeweilige Applikation ausgewählt. Für hohe Abtragsleistung haben sich Siliciumkarbidmaterialien bewährt, wobei mit Partikelgrößen zwischen 50 und 80 µm gute Ergebnisse erzielt werden.

Die kleineren Abrasivpartikel 60 dienen langsamerem Abtrag und damit einer Glättung der bearbeiteten Oberfläche, und ihr Durchmesser kann grob etwa die Hälfte oder ein Drittel des Durchmessers der großen Abrasivpartikel 56 betragen. Auch kann man für diese Abrasivpartikel Materialien auswählen, die weniger starke Schneidkanten ausbilden als Siliciumkarbidmaterialien. Beispiele für derartige Materialien sind z. B. Aluminiumkeramikmaterialien. In der Praxis bewährt haben sich z. B. plättchen- oder scheibenförmige Aluminiumkeramikpartikel mit einem Durchmesser um 25 µm, wobei diese Partikel sich zugleich durch hydrophile Eigenschaften ihrer Oberfläche auszeichnen. Hydrophile Partikeloberflächen sind im Hinblick auf die Herstellung gut handhabbarer stabiler Aufschlämmungen in Wasser von Vorteil.

Die Versiegelungspartikel 60 haben kleinen Durchmesser. Ihr Durchmesser wird im Hinblick auf den Durchmesser von Dentinkanälchen des Zahnmateriales gewählt, welche durch die Versiegelungspartikel 60 verschlossen werden sollen, wie nachstehend noch genauer beschrieben werden wird. In der Praxis liegt der Durchmesser dieser Versiegelungspartikel unter 3 µm, vorzugsweise bei etwa 1µm. Geeignete Materialien für die Versiegelungspartikel 60 sind insbesondere wieder Aluminiumkeramikmaterialien. Bei den Versiegelungspartikeln wird bei der Herstellung wieder auf scharfkantige Oberfläche geachtet. Zum einen deshalb, um diesen Partikeln trotz ihres geringen Durchmessers noch brauchbare abrasive Eigenschaften zu geben, da diese Partikel zugleich auch zum Abtragen von Material dienen sollen, zum anderen deshalb, um ein möglichst zuverlässiges mechanisches Verhaken der Versiegelungspartikel an den Enden der Dentinkanälchen und an Unregelmäßigkeiten der Oberfläche der bearbeiteten Kavität zu gewährleisten. Auf diese Weise bilden die Versiegelungspartikel Haftstellen, an denen sich Füllmaterial verhaken und gut anlagern kann.

Bezüglich der Mengenverhältnisse, in denen die großen Abrasivpartikel 56, die kleineren Abrasivpartikel 58 und die Versiegelungspartikel 60 vorliegen, wird auf die Ausführungen in der Beschreibungseinleitung hingewiesen.

Die obigen Angaben für Partikelgrößen sind die im Einsatz beim Materialabtrag gewünschten Partikelgrößen. Bestehen die Partikel aus sprödem, unter Ultraschalleinwirkung zerbrechendem Material, so können die Größen der Partikel, die sich im zugeführten Arbeitsmedium befinden, größer sein, wenn sich nur an der Arbeitsstelle durch Zerbrechen dieser Partikel die gewünschten Partikelgrößen ergeben.

Im Hinblick auf hohe Abtragsleistung ist es wünschenswert, wenn die großen Abrasivpartikel 56, die mittleren Abrasivpartikel 58 und die feinen abrasiven Versiegelungspartikel 60 jeweils in sich nur geringe Streuung der Korngröße um den gewünschten mittleren Durchmesser aufweist.

In dem Mischbehälter 52 dreht sich ein Mischstab 62 aus magnetischem Material, der durch das von einer Magnetspule 64 erzeugte Drehfeld bewegt wird.

Bei dem in Figur 1 gezeigten Präparationsgerät ist das Werkzeug 36 hohl und über einen Schlauch 164 mit dem Ausgang einer zweiten Pumpe 166 verbunden, die aus einem Vorratsbehälter 168 ein abrasives Behandlungsmedium 170 ansaugt. Letzteres besteht in der Praxis wieder aus Wasser, großen Abrasivpartikeln 172 und beim hier betrachteten Ausführungsbeispiel zusätzlich aus zum Verstopfen der Dentinkanäle 74 dienenden Versiegelungspartikeln 174. Dadurch, daß im Behandlungsmedium 170 keine mittleren Durchmesser aufweisenden Abrasivpartikeln 58 (werden weiter unten besprochen) entsprechende Partikel gezeigt sind, soll angedeutet werden, daß sich dieses Behandlungsmedium 170 von einem weiter unten besprochenene Behandlungsmedium 48 unterscheiden kann. Diese Unterscheidung kann auch bezüglich weiterer Zusätze zum Behandlungsmedium bestehen, die zur Einstellung der Viskosität dienen oder medikamentöse Funktion haben.

Durch einen Doppelpfeil in der Ansaugleitung der Pumpe 166 ist angedeutet, daß man diese Pumpe auch dazu verwenden kann verbrauchtes Arbeitsmedium über das Werkzeug 36 abzusaugen.

Im unteren rechten Teil von Figur 1 ist bei 64 der koronale Abschnitt eines Zahnes 66 mit seiner Schmelzschicht 68, seinem Dentinvolumen 70 und der Zahnhöhle 72 wiedergegeben. Von der Zahnhöhle 72 erstrecken sich die kleinen Durchmesser aufweisenden Dentinkanäle 74 durch das Dentinvolumen 70 zur Schmelzschicht 68.

In dem Zahn 66 ist von der Kaufläche her eine Vertiefung 76 erzeugt worden, welche sich durch die Schmelzschicht 68 in das Dentinvolumen 70 hineinerstreckt. Das Abtragen der entsprechenden Materialmenge erfolgte dadurch, daß man unter Abgabe von abrasivem Behandlungsmedium 48 durch das Abgaberohr 46 und gleichzeitiger Ultraschallbeaufschlagung des Werkzeuges 36 die untere Stirnfläche und die Seitenflächen des Werkzeuges 36 gegen das Zahnschmelzmaterial bzw. Dentinmaterial drückte. Dabei wird von der schwingenden Werkzeugoberfläche den abrasiven Partikeln eine hohe Geschwindigkeit erteilt, und wenn diese Partikel auf die gegenüberliegende Materialoberfläche auftreffen, lösen sie aus dieser kleine Bruchstücke heraus. Diese Art des Materialabtrages ist schonend und führt zu keinen größeren Gewebebeschädigungen, insbesondere keinen Rissen. Durch entsprechendes Führen des Werkzeuges in den drei Koordinatenrichtungen und Drehen des Handstückes um die den Koordinatenrichtungen entsprechenden Achsen (diese Bewegungen sind symbolisch bei 78 angedeutet) kann der Arzt die jeweils erforderliche Zahnkavität erzeugen.

Figur 1 zeigt den Zustand beim Abschluß der Präparation der Kavität, und man erkennt, daß die beim Erzeugen der Kavität freigelegten Enden von Dentinkanälen 74 durch einige der feinen Versiegelungspartikel 60 verschlossen sind. Dies vermindert die Permeabilität des an der Kavität angrenzenden Dentinvolumens im Sinne einer als Unterfüllung wirkenden Isolationsschicht.

Figur 3 zeigt eine Weiterbildung der Erfindung, bei welcher ein Formwerkzeug durch den später mit dem Zahnmaterial zu verbindenden Form-Füllkörper selbst gebildet ist.

In das obere Ende eines Form-Füllkörpers 176, dessen Geometrie der des Formwerkzeuges 86 im wesentlichen entspricht, ist ein Befestigungsteil 178 eingegossen, welches auf das Montageteil 38 paßt. Der Form-Füllkörper 176 ist in seinem oberen Abschnitt mit einer Sollbruchstelle 180 ausgebildet, so daß man nach dem Einsenken des Form-Füllkörpers 176 (unter gleichzeitiger Zufuhr von abrasivem Behandlungsmedium, wie oben beschrieben) das Befestigungsteil 180 abbrechen bzw. durch Schlag absprengen kann. Der Form-Füllkörper 176 wird dann mit dem Zahnmaterial verklebt, wie oben beschrieben, und der obere Abschnitt des Form-Füllkörperes 176 wird zur Wiederherstellung der Kaufläche abgetragen.

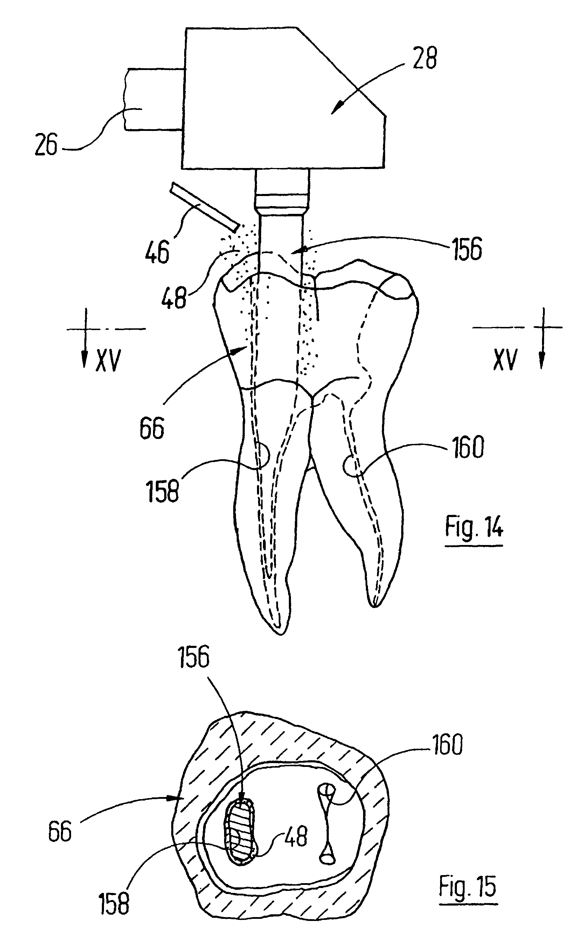

Untenstehend wird unter Bezugnahme auf Figur 14 noch ein weiteres Beispiel für einen Form-Füllkörper, der zugleich auch als Formwerkzeug dient, beschrieben werden, das zur Behandlung von Zahnwurzelkanälen dient.

Für derartige zugleich Werkzeuge darstellende Form-Füllkörper geeignete Materialien sind Metallegierungen, z. B. Titanlegierungen, sowie oxidische oder nichtoxidische Keramiken, insbesondere Aluminiumoxid-Keramik oder Siliciumkarbid-Sintermaterialien.

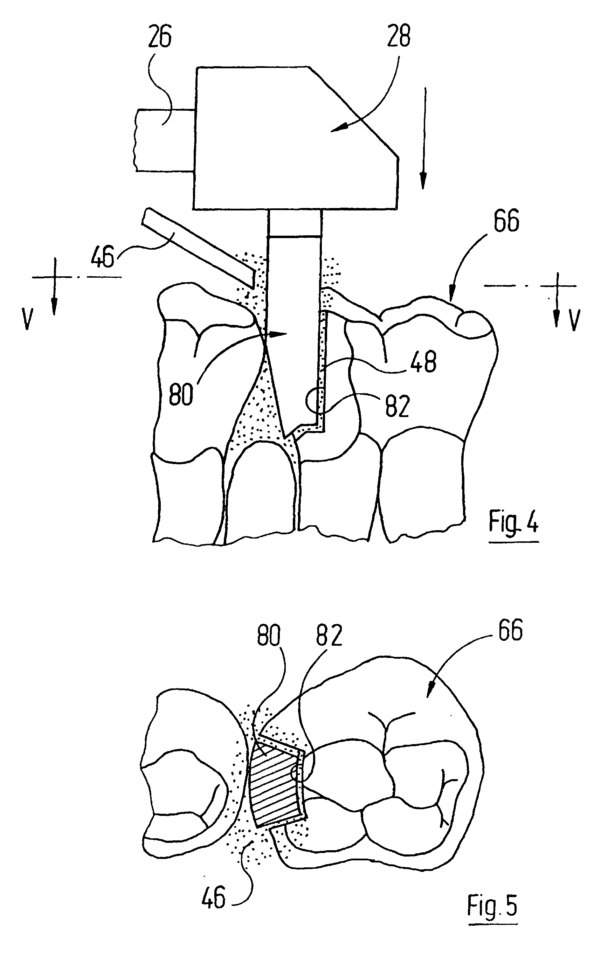

Anhand der Figuren 4-9 wird nun die Versorgung eines größeren kariösen Zahndefektes beschrieben.

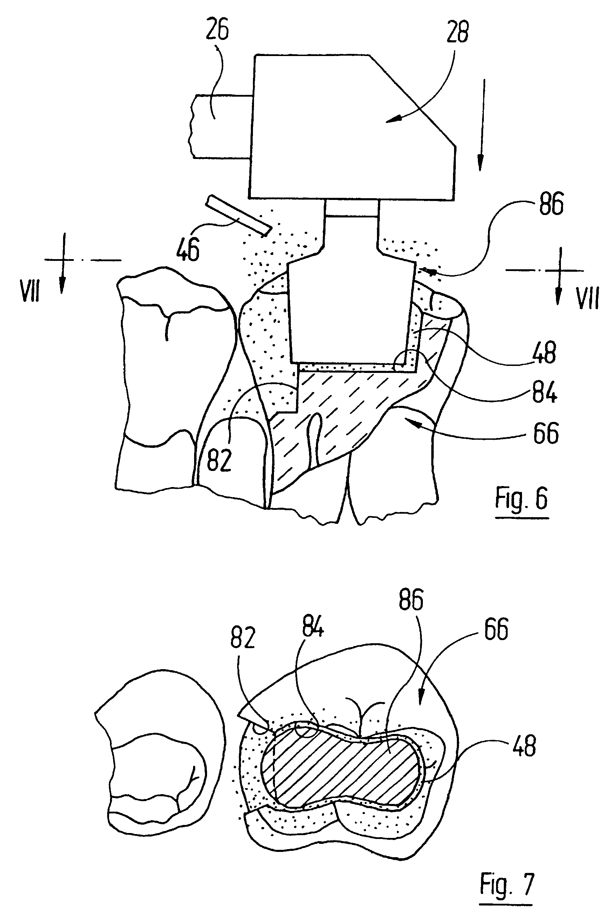

In einem ersten Behandlungsschritt, der in den Figuren 4 und 5 gezeigt ist, wird ein seitlicher kariöser Bereich des Zahnes unter Verwendung eines ersten Formwerkzeuges 80 entfernt, das an dem Umlenkkopf 28 befestigt ist. Dieses Werkzeug hat in Querschnittsansicht gesehen die Form eines gekrümmten Trapezes und in axialer Schnittansicht gesehen die Form eines Keiles. Durch rein axiales Vorschieben des Formwerkzeuges 80, welches über den Umlenkkopf 28 mit Ultraschall beaufschlagt ist, bei gleichzeitigem Zuführen abrasiven Behandlungsmediums 48 ist der kariöse Randbereich des Zahnes abgetragen worden, wobei eine zur Außenform des Formwerkzeuges 80 komplementäre Ausnehmung 82 im Zahn 66 erzeugt wurde. Gleichzeitig können die Kavitätenränder z. B. durch Ausformung einer partiellen Randanschrägung definitiv ausgeformt werden.

Im zweiten Behandlungsschritt, welcher in den Figuren 6 und 7 gezeigt, wird im Zahn 66 eine mittige Ausnehmung 84 erzeugt, deren Randkontur etwa die einer liegenden Acht hat. Hierzu wird ein weiteres Formwerkzeug 86 mit entsprechendem Querschnitt in axialer Richtung vorgeschoben, wobei wiederum abrasives Behandlungsmedium 48 zugeführt wird, welches sich zwischen die Außenfläche des Formwerkzeuges 86 und das Zahnmaterial legt.

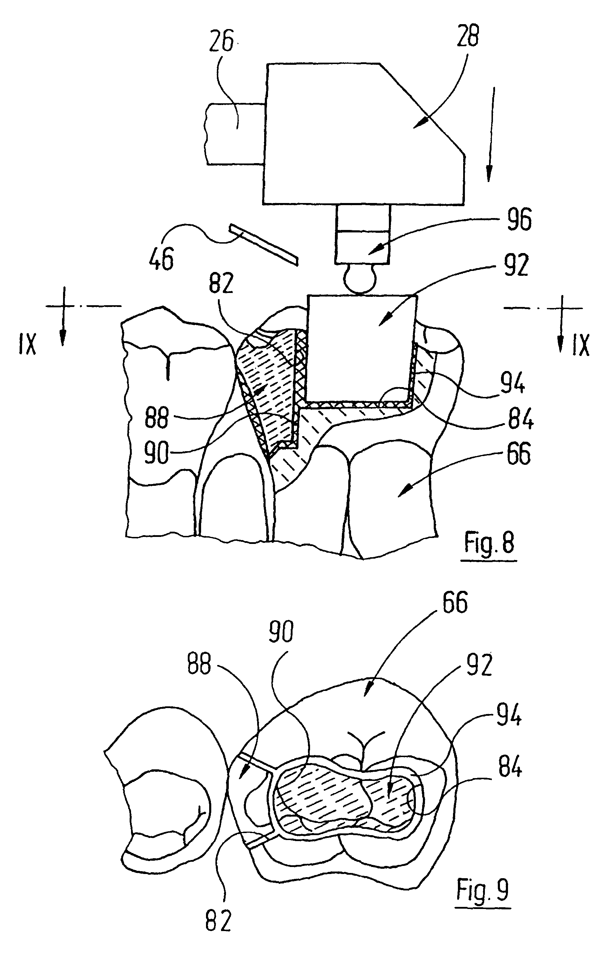

Figur 8 zeigt, daß anschließend an das Erzeugen der beiden Ausnehmungen 82 und 84 in die Ausnehmung 82 ein erster Form-Füllkörper 88 eingesetzt worden ist, der aus Zahnersatzmaterial besteht und dessen Umfangskontur derjenigen des Formwerkzeuges 80 entspricht. Der Formkörper 88 ist über eine Fugen-Kompositschicht 90 mit dem Zahnmaterial verbunden. Die obere Stirnfläche des Form-Füllkörpers 88 ist unter Verwendung eines Bearbeitungswerkzeuges, welches in der Praxis demjenigen nach Figur 1 ähneln kann, so bearbeitet, daß sie die ursprüngliche Kaufläche nachbildet.

In die mittige Ausnehmung 84 wird gerade ein weiterer Form-Füllkörper 92 eingesetzt. Dessen Außenkontur entspricht der Außenkontur des Formwerkzeuges 86. Zwischen der Außenfläche des Form-Füllkörpers 92 und den Wandflächen der Ausnehmung 84 ist eine weitere Fugen-Kompositschicht 94 gezeigt, die noch nicht abgebunden hat. Durch ein Drück-Werkzeug 96, welches nun am Umlenkkopf 28 des Handstückes 10 angebracht ist, wird auf das obere Ende des Form-Füllkörpers 92 Ultraschall übertragen, wodurch das Einführen des Form-Füllkörpers 92 in die Ausnehmung 84 unterstützt wird, da die Viskosität des Fugen-Kompositmateriales durch die Vibration herabgesetzt wird. Diese Art des Einsetzens des Form-Füllkörpers ist im Hinblick auf möglichst dünne Fugen-Komposit- oder Klebstoffschichten von Vorteil.

In Figur 8 ist der Standard-Form-Füllkörper 92, der vom Zahnarzt zur Verwendung in durch das Formwerkzeug 86 erzeugten Ausnehmungen bereitgehalten wird, noch in seiner unveränderten Geometrie wiedergegeben. Nach dem Abbinden der Fugen-Kompositschicht 94 müssen die über die Kaufläche überstehenden Abschnitte des Form-Füllkörpers 92 so entfernt werden, daß die entstehende Oberseite des Form-Füllkörpers 92 wieder die ursprüngliche Kaufläche wiedergibt. Die entsprechenden Konturen sind in Figur 9 durch dünne Linien angedeutet.

Bei der oben stehend unter Bezugnahme auf Figur 8 gegebenen Beschreibung der restaurativen Behandlungsphase wurde der besseren Erläuterung der Erfindung halber davon ausgegangen, daß zuerst der erste Form-Füllkörper 88 eingesetzt und seinem Platz durch Kompositmaterial fixiert wird, dann seine obere Stirnfläche gemäß der gewünschten Kauflächengeometrie bearbeitet wird und anschließend der Form-Füllkörper 92 in der Kavität angebracht wird. Es versteht sich, daß bei diesem Vorgehen die Kompositschicht zwischen den beiden Form-Füllkörpern erst beim Einsetzen des Form-Füllkörpers 92 angebracht wird, damit die beiden Form-Füllkörper gut miteinander verbunden werden.

In der Praxis wird man in der Regel in der Regel so vorgehen, daß man beide Form-Füllkörper 88 und 92 in einem gemeinsamen Arbeitsgang unter Verwendung von Kompositmaterial in der Kavität anbringt und die Bearbeitung der oberen Stirnfläche beider Form-Füllkörper zur Herstellung der gewünschten Kauflächengeometrie gemeinsam vornimmt.

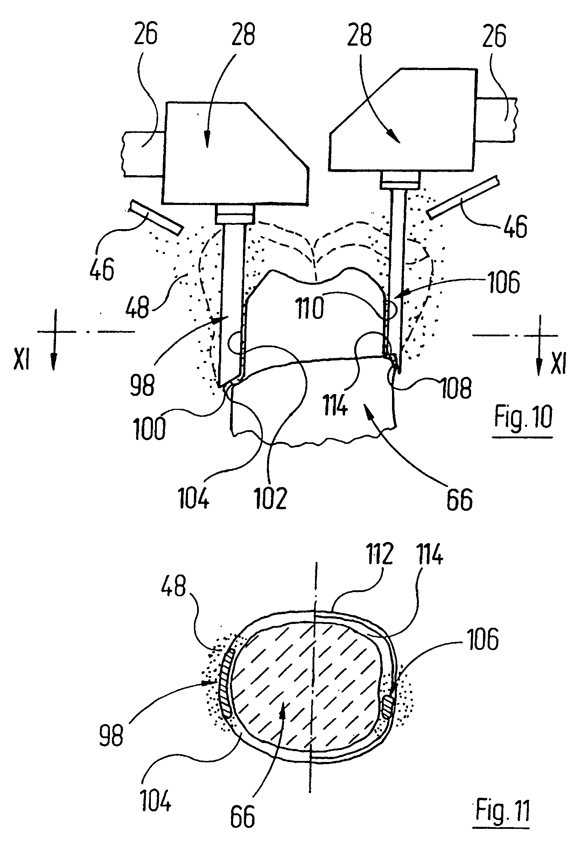

Die Figuren 10 und 11 zeigen die Verwendung von Ultraschall-Werkzeugen bei der Präparation von Zahnstümpfen für das spätere Aufsetzen einer Krone (rechte Seite: Hohlkehl-Präparation; linke Seite: Präparation einer abgeschrägten Stufe). Dieses Bearbeiten erfolgt wiederum unter gleichzeitigem Zuführen eines abrasiven Behandlungsmediums 48.

In der linken Hälfte der Figuren 10 und 11 ist ein Werkzeug 98 gezeigt, welches im Vertikalschnitt gesehen die Form eines Rechteckes, in transversalem Schnitt gesehen die Form eines gebogenen niederen Rechteckes mit abgerundeten Enden hat. Die Querschnittskrümmung ist im Hinblick auf die Zahnkrümmung im zu behandelnden Bereich gewählt.

In der Praxis hat der Zahnarzt mehrere derartige Bearbeitungswerkzeuge mit unterschiedlicher Krümmung in seinem Werkzeugsatz.

Wie Figur 10 zeigt, ist die untenliegende Stirnfläche 100 des Werkzeuges 98 schräg abfallend, so daß dieses Werkzeug am Zahnstumpf eine vertikale Seitenfläche 102 erzeugt, die unten durch eine schräg nach außen abfallende Hohlkehle 104 begrenzt ist.

Es versteht sich, daß die Geometrie der hergestellten Form im horizontalen Durchmesser und im vertikalen Durchmesser auch anders verknüpft sein kann: es kann auch mit dem kleinen Durchmesser eine Hohlkehle und mit dem größeren Durchmesser eine abgeschrägte Stufe erzeugt werden.

Das in den rechten Hälften der Figuren 10 und 11 gezeigte Werkzeug 106 ähnelt dem Werkzeug 98, es hat jedoch nur kleine Umfangserstreckung, so daß es sich für die Behandlung von Zahnstümpfen unterschiedlichster Umfangskrümmung allgemein einsetzen läßt. Die untere Stirnfläche 108 dieses Werkzeuges 106 ist z.B. abgestuft: sie hat einen senkrecht zur Werkzeugachse stehenden Stirnflächenabschnitt und einen sich hieran anschließenden, nach unten abfallenden Stirnflächenabschnitt. Auf diese Weise erzeugt das Werkzeug 106 am Zahnstumpf wiederum eine vertikale Seitenfläche 110 und eine nach außen abfallende Anschrägung 112, wobei jedoch zwischen der Anschrägung 112 und der Seitenfläche 110 noch eine horizontale Stufe 114 erzeugt wird.

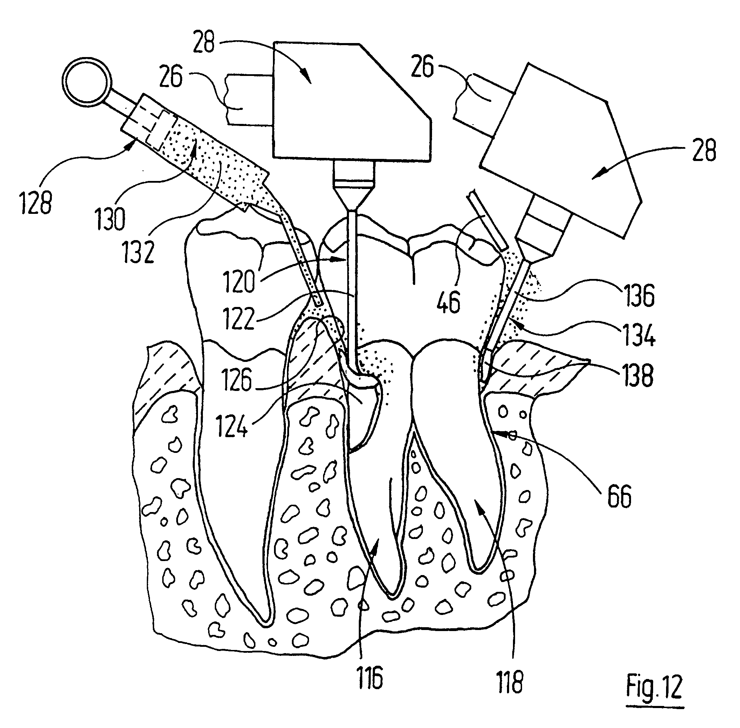

Figur 12 zeigt das schonende Abtragen von Material, insbesondere supra- oder subgingivalem Zahnstein, Wurzelkonkrementen und/oder Plaque am zervikalen Ende von Zahnwurzeln 116, 118. Ein erstes Werkzeug 120 hat einen Stiel 122, der ähnlich aussehen kann wie der des Werkzeuges 106, und am unteren Ende des Stieles 122 ist ein flächiges Arbeitsteil 124 angebracht, welches im wesentlichen die Form einer Pflanzschaufel hat, also in Umfangsrichtung gemäß der Krümmung der Zahnwurzel 116 gekrümmt ist. Das Werkzeug 120 ist in eine Zahnfleischtasche 126 eingeführt, die aus einer Spritze 128 mit Behandlungsmedium 130 gefüllt wird. Letzteres hat ein höher viskoses flüssiges Grundmaterial, in welchem wieder Abrasivpartikel 132 verteilt sind und welches darüber hinaus gegebenenfalls noch chemische Ätzmittel und/oder keimtötende Substanzen enthalten kann.

In Abwandlung kann man auch ein Behandlungsmedium 130 verwenden, welches keine Abrasivpartikel enthält.

Ferner sei darauf hingewiesen, daß die Werkzeuge 120 und 134 anders als klassische Curetten keine geometrisch definierte Schneide aufzuweisen brauchen.

Im rechten Teil von Figur 12 ist ein der Zahnsteinentfernung dienendes Werkzeug 134 für die Behandlung der Außenfläche der Zahnwurzel 118 wiedergegeben, welches die Form eines Golfschlägers oder Hockeyschlägers hat. Das Werkzeug 134 hat somit einen Stiel 136, der ein schmales transversales Arbeitsteil 138 trägt. Das Werkzeug 134 eignet sich gut zur Behandlung von auch in Längsrichtung gekrümmten Zahnwurzeln oder von Zahnwurzeln mit in Umfangsrichtung konkav gekrümmten Oberflächen.

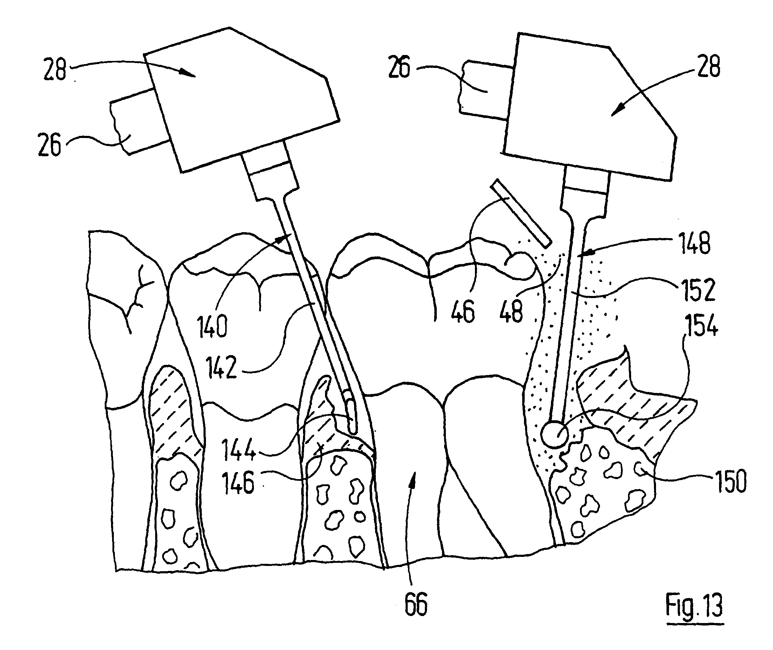

Figur 13 zeigt im linken Teil ein Werkzeug 140 (Ultraschall-Skalpell), welches zum Abtragen von Weichgewebe, insbesondere Zahnfleisch, oder zur Präparation von Weichgewebsinzissionen dient. An einem Stiel 142 ist ein transversales Arbeitsteil 144 befestigt, welches aus einem Material hergestellt ist, das Ultraschallenergie in Wärme umsetzt. Ein geeignetes derartiges Material ist z.B. ein aus zwei oder mehr unterschiedlichen Materialschichten aufgebautes Verbundmaterial. Beispiele hierfür sind ein Bimetallstreifen oder mit einer keramischen Beschichtung versehene Metalle. Beispiele für im Volumen hohe innere Reibung aufweisende Materialien sind nichtrostende Stähle. Das Werkzeug 140 arbeitet somit als Ultraschall-Kauter und kann von dem bei 146 gezeigten Zahnfleisch sehr genau steuerbar Inzissionen anlegen, Teile abtragen oder Gefäße verkauten.

Im rechten Teil von Figur 13 ist ein Knochenpräparations-Werkzeug 148 zum Abtragen von Knochenmaterial 150 wiedergegeben. Von einem stabförmigen Stiel 152 ist ein z.B. kugelförmiges Arbeitsteil 154 getragen. In Abwandlung kann das Arbeitsteil 154 auch etwa die Form einer V-förmigen oder pyramidenförmigen Meißelspitze haben. Das im rechten Teil von Figur 13 gezeigte Werkzeug 148 wird vorzugsweise wieder unter gleichzeitiger Zuführung von abrasivem Behandlungsmedium 48 betrieben.

Figur 14 zeigt ein Werkzeug 156, welches zur Präparation eines Zahnwurzelkanales 158 dient. Dieser ist schon auf herkömmliche Weise oder unter Verwendung eines Werkzeuges, wie es obenstehend unter Bezugnahme auf die Figuren 1-7 beschrieben wurde, freigelegt und weitgehend unter Verwendung herkömmlicher Werkzeuge erweitert worden. Das Werkzeug 156 dient zur schonenden Rest-Bearbeitung des Zahnwurzelkanales 158 und hat hierzu einen transversalen Querschnitt, der der Form eines Rechteckes mit abgerundeten Ecken oder eines Ovals entspricht, wobei der transversale Querschnitt zum freien Ende des Werkzeuges 156 hin abnimmt. In Längsrichtung gesehen hat das Werkzeug 156 die Form eines leicht gekrümmten Keiles.

Das Werkzeug 156 ist einem Satz von Werkzeugen entnommen, deren einzelne Geometrien den durchschnittlichen Standardgeometrien (Form, Größe, Durchmesser) von Wurzelkanälen der verschiedenen Zähne bzw. Zahngruppen nachempfunden ist.

In Abwandlung des in Figur 14 gezeigten Ausführungsbeispieles kann man ein Werkzeug 156 zur Präparation eines Zahnwurzelkanales auch mit einer Sollbruchstelle oder Trennmarkierung versehen, so daß der zum bearbeiteten Wurzelkanal komplementäre Abschnitt des Werkzeuges nach Aufbrechen der Sollbruchstelle oder Abflexen an der Trennmarkierung als Restaurationsteil in dem behandelten Wurzelkanal verankert werden kann. Dieser Abschnitt des Werkzeuges 156, der die Form eines stiftähnlichen Form-Füllkörpers bildet, kann dann als Verankerung für Aufbauteile dienen. Als Material für derartige Werkzeuge kommen Keramikmaterialien, für Zahnprotesen oder Implantate verwendete Metalle und auch Kunststoffe in Frage.

Der rechte Teil der Figuren 14 und 15 zeigt einen nicht präparierten Zahnwurzelkanal 160.

## Patentansprüche

1. Vorrichtung zur Ultraschallpräparation von menschlichem oder tierischem Hart- oder Weichgewebe oder von Zahn- oder Knochenersatzmaterialien mit einem Ultraschall-Schwingungsgenerator (16) sowie einem durch diesen angetriebenen Werkzeug (36), bei der zwischen dem Schwingungsgenerator (16) und dem Werkzeug ein Schall-Umlenkkopf (28) vorgesehen ist, der ein parallel zur Längsachse (H) des Schwingungsgenerators (16) bewegliches getriebenes Eingangselement (33) und ein treibendes Ausgangselement (34) aufweist, wobei letzteres längs einer Achse (W) beweglich ist, die mit der Achse des Schwingungsgenerators einen Winkel von 60° bis 120 ° bildet, und bei der das Werkzeug (36) mit dem treibenden Ausgangsteil (34) des Umlenkkopfes (28) verbunden ist, wobei der Umlenkkopf (28) einen Schwingkörper aufweist, der die Form eines Ringes, einer Scheibe oder einer Hohlkugel hat und der eine mit der Arbeitsfrequenz des Schwingungsgenerators (16) übereinstimmende Resonanzfrequenz aufweist, wobei der Durchmesser des Schwingkörpers kleiner als 30 mm, vorzugsweise kleiner als 20 mm ist.

2. Vorrichtung nach Anspruch 1, **dadurch gekennzeichnet, daß** das Werkzeug (36; 80; 82; 96; 98; 106; 120; 134; 140; 148; 156; 176) parallel zu seiner Längsachse schwingt.

3. Vorrichtung nach Anspruch 2, **dadurch gekennzeichnet, daß** der Winkel zwischen der Längsachse (H) des Schwingungsgenerators (16) und der Längsachse (W) des Werkzeuges (36; 80; 86; 96; 98; 106; 120; 134; 140; 148; 156; 176) 90° beträgt.

4. Vorrichtung nach einem der Ansprüche 1-3, **dadurch gekennzeichnet, daß** das Gesamtsystem, welches durch den Schwingungsgenerator (16), den Umlenkkopf (28) und das Werkzeug (36; 80; 86; 96; 98; 106; 120; 134; 140; 148; 156; 176) gebildet ist, in Resonanzfrequenz oszilliert.

5. Vorrichtung nach einem der Ansprüche 1-4, **dadurch gekennzeichnet, daß** das Werkzeug (80; 86; 156; 176) die Form eines Form-Füllkörpers (88; 92; 176) darstellt, der aus Gewebeersatzmaterial, z.B. Zahnersatzmaterial, hergestellt ist, und das Werkzeug (80; 86; 156; 176) und der Umlenkkopf (28) so ausgebildet sind, daß das Werkzeug (80; 86; 156; 176) unter Oszillation wenigstens teilweise in das zu bearbeitende Material eingesenkt werden kann.

6. Vorrichtung nach Anspruch 5, **gekennzeichnet durch** einen Satz unterschiedlicher Werkzeuge (80; 86; 156; 176) welche für unterschiedliche Zahngrößen und/ oder unterschiedliche Mindespräparationstiefen, die z.B. spezifisch für das verwendete Restaurationsmaterial sind, ausgelegt sind und Negativformen entsprechend unterschiedlicher Form-Füllkörper (88; 92; 176) darstellen.

7. Vorrichtung nach Anspruch 6, **gekennzeichnet durch** einen Satz an Form-Füllkörpern (88; 92; 176), der in gleichen Formen und Größenabstufungen vorliegt wie die Werkzeuge (80; 86; 156; 176) die zur Kavitätenpräparation verwendet werden.

8. Vorrichtung nach Anspruch 6 oder 7, **dadurch gekennzeichnet, daß** die Form-Füllkörper (88; 92; 176) aus Keramikmaterial oder Polymer-Kompositmaterial oder einem Metall, z. B. Titan, bestehen und ihre Oberflächen zumindest teilweise mit Befestigungsmaterialien, insbesondere Polymeradhäsiven oder Zementen benetzt oder form- oder kraftschlüssig, verbunden sind.

9. Vorrichtung nach einem der Ansprüche 1-8, **dadurch gekennzeichnet, daß** das Werkzeug (140) ein Arbeitsteil (144) umfaßt, welches aus einem Material besteht, das sich unter Ultraschalleinwirkung erhitzt.

10. Vorrichtung nach Anspruch 9, **dadurch gekennzeichnet, daß** sich das Arbeitsteil (144) des Werkzeuges (140) durch Reibungswärme erhitzt, insbesondere innere Reibungswärme, wozu das Arbeitsteil vorzugsweise aus mehreren unterschiedlichen Materialschichten besteht.

11. Vorrichtung nach einem der Ansprüche 1-10, **gekennzeichnet durch** wenigstens eine Einrichtung (46, 50, 52; 164, 166, 168) zum Zuführen von abrasivem Behandlungsmedium (48; 170) zum Werkzeug (36; 80; 86; 98; 106; 120; 134; 148; 156; 176), wobei vorzugsweise das abrasive Behandlungsmedium (48; 170) kleine abrasive Versiegelungspartikel (60) mit einem mittleren Teilchendurchmesser von weniger als 5µm enthält.

12. Vorrichtung nach Anspruch 11, **dadurch gekennzeichnet, daß** die abrasiven Versiegelungspartikel (60) eine mittlere Teilchengröße um etwa 1µm aufweisen.

13. Vorrichtung nach Anspruch 11 oder 12, **dadurch gekennzeichnet, daß** unter den abrasiven Partikel Versiegelungspartikel (60) sind, die aus oxidischen und/oder silikatischen und/oder karbidischen und/oder nitridischen und/oder polymerhaltigen Materialien bestehen und die Oberfläche der Versiegelungspartikel zumindest teilweise mit Silanen und/ oder Polymeren einen wenigstens teilweise chemischen Verbund eingehen kann und/oder wenigstens teilweise unter Einwirkung wenigstens teilweise säurehaltiger gelierender Fugen-Füllmaterialien, insbesondere durch Lichteinwirkung- oder chemisch härtender Glasionomerzemente oder Kompomere, in ein sich bildendes Gel mit einbezogen wird, wobei vorzugsweise ein Volumenanteil Versiegelungspartikel auf 2 bis 20, vorzugsweise etwa 10 Volumentanteile Abrasivpartikel eingesetzt wird.

14. Vorrichtung nach einem der Ansprüche 11-13, **dadurch gekennzeichnet, daß** das Behandlungsmedium (130) die Form wenigstens eines Gels hat.

15. Vorrichtung nach Anspruch 14, **dadurch gekennzeichnet, daß** das Behandlungsmedium (130) eine etwa durch Zugabe von Aerosil oder gelierenden Trägersubstanzen, z.B. Gelatine, veränderte Viskosität aufweist und daß sich das gelförmige Behandlungsmedium unter Ultraschalleinwirkung wenigstens teilweise verflüssigt.

16. Vorrichtung nach Anspruch 14 oder 15, **dadurch gekennzeichnet, daß** die Basiskomponente des Behandlungsmediums (130) Glyceringel oder etwa 1-10%iges Chlorhexidingel oder Gelatine ist.

17. Vorrichtung nach einem der Ansprüche 5-16, **dadurch gekennzeichnet, daß** das Werkzeug und ein in eine Gewebekavität einzusetzender Form-Füllkörper gemeinsam durch einen aus gewebeverträglichem hartem Material hergestellten Form-Füllkörper (156; 176) gebildet sind.

18. Vorrichtung nach Anspruch 17, **dadurch gekennzeichnet, daß** der Form-Füllkörper (156; 176) aus oxidkeramischen oder nichtoxidkeramischen Werkstoffen oder aus metallischem Material, z. B. Titan, besteht.

19. Vorrichtung nach Anspruch 17 oder 18, **dadurch gekennzeichnet, daß** der Form-Füllkörper (176) ein mit dem treibenden Ausgangsteil (34; 162d) des Umlenkkopfes (28) verbindbares Befestigungsteil (178) aufweist, welches über eine Sollbruchstelle (180) mit dem in die Gewebekavität einzusetzenden Abschnitt des Form-Füllkörpers (176) verbünden, z. B. verschraubt, verklemmt oder verlötet ist.

20. Vorrichtung nach einem der Ansprüche 11-19, **dadurch gekennzeichnet, daß** das Behandlungsmedium (48; 170) Abrasivpartikel (56) enthält, deren Durchmesser vorzugsweise etwa dem Gesamthub der Ultraschall-Schwingung des Werkzeuges (36; 80; 86; 98; 106; 120; 134; 148; 156; 176) entspricht, wobei vorzugsweise ein Volumentanteil Abrasivpartikel auf 3 bis 50, wiederum vorzugsweise auf 5 bis 20 und nochmals vorzugsweise eta 10 Volumenteile Flüssigkeit eingesetzt wird.

21. Vorrichtung nach einem der Ansprüche 11-20, **dadurch gekennzeichnet, daß** die abrasiven Partikel (56, 58, 60) eine hydrophile Oberfläche oder eine hydrophile Oberflächenbeschichtung aufweisen.

22. Vorrichtung nach einem der Ansprüche 11-21, **gekennzeichnet durch** eine weitere Einrichtung (46, 50, 52) zum Zuführen von Behandlungsmedium (48) zur Außenseite des Werkzeuges (36; 80; 86; 98; 106; 120; 134; 148; 156; 176).

23. Vorrichtung nach einem der Ansprüche 1-22, **dadurch gekennzeichnet, daß** das Werkzeug (36; 80; 86; 96; 106; 120; 134; 148; 156) wenigstens partiell hohl ist und mit einer Einrichtung (164, 166, 168) zum Zuführen von abrasivem Behandlungsmedium (170) verbunden ist.

24. Vorrichtung nach einem der Ansprüche 1-23, **dadurch gekennzeichnet, daß** das Werkzeug (96) wenigstens einen balligen Vibratorkopf aufweist.

25. Vorrichtung nach einem der Ansprüche 1-23, **dadurch gekennzeichnet, daß** das Werkzeug (86; 98; 120; 138; 176) einen gekrümmten transversalen Querschnitt aufweist.

26. Vorrichtung nach einem der Ansprüche 1-23, **dadurch gekennzeichnet, daß** das Werkzeug (120) ein rinnenförmiges oder schaufelförmiges Arbeitsteil (124) aufweist.

27. Vorrichtung nach einem der Ansprüche 1-23, **dadurch gekennzeichnet, daß** die freie Stirnfläche (100: 108) des Werkzeuges (98; 106) einen zur Werkzeuglängsachse schräg verlaufenden oder im Sinne einer Hohlkehle gekrümmten Abschnitt aufweist.

28. Vorrichtung nach Anspruch 27, **dadurch gekennzeichnet, daß** die freie Stirnfläche (108) des Werkzeuges (106) einen senkrecht zur Werkzeuglängsachse verlaufenden Abschnitt aufweist.

29. Vorrichtung nach einem der Ansprüche 1-23, **dadurch gekennzeichnet, daß** das Werkzeug (134) einen Stiel (136) und ein an dessen freiem Ende angebrachtes transversales Arbeitsteil (138) aufweist.

30. Vorrichtung nach einem der Ansprüche 1-23, **dadurch gekennzeichnet, daß** das Werkzeug (148) eine Meißelspitze (154) aufweist.

31. Vorrichtung nach einem der Ansprüche 1-23, **dadurch gekennzeichnet, daß** das Werkzeug (156) eine der Geometrie eines Zahnwurzelkanales (158) entsprechende Geometrie hat.

32. Vorrichtung nach einem der Ansprüche 1-31, **dadurch gekennzeichnet, daß** das Werkzeug einen bezogen auf seine Längsachse transversal schwingenden Endabschnitt aufweist.

33. Vorrichtung nach einem der Ansprüche 1-33, **gekennzeichnet durch** mindestens zwei parallel versetzt in das Gewebe einsenkbare unterschiedliche Formwerkzeuge (86; 86), die zusammen die Form einer im Gewebe zu erzeugenden Kavität vorgeben.

## Claims

1. An apparatus for ultrasonic preparation of human or animal hard or soft tissue as well as of dental or bone replacement materials, comprising an ultrasonic vibration generator (16) as well as a tool (36) driven thereby, wherein a sound deflecting head (28) is arranged between the vibration generator (16) and the tool, which comprises a driven input element (33), which is movable in a direction being parallel to the longitudinal axis of the vibration generator (16), as well as a driving output element (34), which is movable along an axis cooperating with the axis of the vibration generator to define an angle being between 60° to 120°, and wherein the tool (36) is connected to the driving output member (34) of the deflecting head (28), the deflection head (28) comprising a vibrating member having the form of a ring, disk or a hollow sphere having a resonance frequency coinciding with the operating frequency of the vibration generator (16), the diameter of the vibrating member being smllaer than 30 mm, perferably smaller than 20 mm.

2. The apparatus in accordance with claim 1, **characterized in that** the tool (36; 80; 82; 96; 98; 106; 120; 134; 140; 148; 156; 176) vibrates in a direction being parallel to its longitudinal axis.

3. The apparatus in accordance with claim 2, **characterized in that** the angle included between the longitudinal axis (H) of the vibration generator (16) and the longitudinal axis (W) of the tool (36; 80; 86; 96; 98; 106; 120; 134; 140; 148; 156; 176) is 90°.

4. The apparatus has in one of claims 1 to 3, **characterized in that** the overall system formed by the vibration generator (16), the deflecting head (28) and the tool (36; 80; 86; 96; 98; 106; 120; 134; 140; 148; 156; 176) oscillates at resonance frequency.

5. The apparatus in accordance with one of claims 1 to 4, **characterized in that** the tool (80; 86; 156; 176) is the model of a shaped filling member (88; 92; 176) which is made from tissue replacement material, e.g. dental replacement material, and **in that** the tool (80; 86; 156; 176) and the deflecting head (28) are formed such that the tool (80; 86; 156; 176) can be sinked at least partially into the material to be treated while simultaneously oscillating.

6. The apparatus in accordance with claim 5, **characterized by** a set of different tools (80; 86; 156; 176) designed for different sizes of a tooth and/or different minimum preparation depths, which e.g. are specific for the replacement material used, and represent negative models corresponding to different shaped filling members (88; 92; 176).

7. The apparatus in accordance with claim 6, **characterized by** a set of shaped filling members (88; 92; 176) being provided in same shapes and same size gradations as the tools (80; 86; 156; 176) used for preparing cavities.

8. The apparatus in accordance with claim 6 or 7, **characterized in that** the shaped filling members (88; 92; 176) are made from ceramic material or polymer composite material or a metal, e.g. titanium, and that the surfaces thereof are at least partially coated with fixing materials, particularly polymer adhesives or cements or are positively connected by means of a force or by means of geometry.

9. The apparatus in accordance with one of claims 1 to 8, **characterized in that** the tool (140) comprises a work portion (144) consisting of a material which heats when exposed to ultrasonics.

10. The apparatus in accordance with claim 9, **characterized in that** the work portion (144) of the tool (114) is heated by frictional heat, particularly internal frictional heat, the work portion preferably consisting of a plurality of different material layers to this end.

11. The apparatus in accordance with one of claims 1 to 10, **characterized by** at least one means (46, 50, 52; 164, 166, 168) supplying abrasive treatment medium (48; 170) to the tool (36; 80; 86; 98; 106; 120; 134; 148; 156; 176), the abrasive treatment medium (48, 170) preferably containing small abrasive sealing particles (60), the average diameter of the particles being smaller than 5 µm.

12. The apparatus in accordance with claim 11 **characterized in that** the average size of the abrasive sealing particles (60) is about 1 µm.

13. The apparatus in accordance with claim 11 or 12, **characterized in that** among the abrasive particles there are sealing particles (60), which consist of oxide-type and/or silicate-type and/or carbide-type and/or nitride-type and/or polymer containing materials, the surface of the sealing particles being apt to establish an at least partly chemical compound with silanes and/or polymers and/or are apt to at least partly be integrated into a gel when exposed to jellying joint filling material at least partly containing an acid, particularly glass ionomer cements or compomers curing upon exposure to light or chemically curing, preferably one unit volume of sealing particles being used in connection with 2 to 20 preferably about 10 unit volumes of abrasive particles.

14. The apparatus in accordance with one of claims 11 to 13, **characterized in that** the treatment medium (130) is provided in the form of at least one gel.

15. The apparatus in accordance with claim 14, **characterized in that** the treatment medium (130) shows modified viscosity, e.g. by addition of an aerosil a gelling base substance, e.g. gelatine, and **in that** the gel-type treatment medium is at least partly transferred into the liquid state when exposed to ultrasonics.

16. The apparatus in accordance with claim 14 or 15, **characterized in that** the base component of the treatment medium (130) is a glycerine gel or a 1 to 10% chloro-hexidine gel or gelatine.

17. The apparatus in accordance with one of claims 5 to 16, **characterized in that** the tool and a shaped filling member to be inserted into a tissue cavity are both formed by a shaped filling member (156, 176) made from a biocompatible hard material.

18. The apparatus in accordance with claim 17, **characterized in that** the shaped filling member (156; 176) is made from ceramic materials of the oxide type or the non-oxide type or from metallic material, e.g. titanium.

19. The apparatus in accordance with claim 17 or 18, **characterized in that** the shaped filling member (156) comprises a mounting portion (178) connectable to the driving output member (34; 162d) of the deflecting head (28), which is connected to the portion of the shaped filling member (176) to be inserted into the tissue cavity by means of a rated break point (180), said connection being obtained e.g. by screwing, wedging or soldering.

20. The apparatus in accordance with one of claims 11 to 19, **characterized in that** the treatment medium (48; 170) includes abrasive particles (56) the diameter of which preferably approximately corresponds to the overall stroke of the ultrasonic vibration of the tool (36; 80, 86; 98; 106; 120; 134; 148; 156; 176), preferably one unit volume of abrasive particles being used for 3 to 30, preferably 5 to 20 and again preferably about 10 unit volumes of liquid.

21. The apparatus in accordance with one of claims 11 to 20, **characterized in that** the abrasive particles (56, 58, 60) have a hydrophilic surface or a hydrophilic surface coating.

22. The apparatus in accordance with one of claims 11 to 21 **characterized by** further means (46, 50, 52) for supplying treatment liquid (48) to the exterior surface of the tool (36; 80; 86; 98; 106; 120; 134; 148; 156; 176).

23. The apparatus in accordance with one of claims 1 to 22, **characterized in that** the tool (36; 80; 86; 96; 106; 120; 134; 148; 156) is at least partially formed as a hollow member and is connected to means (164, 166, 168) for supplying abrasive treatment medium (170).

24. The apparatus in accordance with one of claims 1 to 23, **characterized in that** the tool (96) comprises at least one convex vibrating head.

25. The apparatus in accordance with one of claims 1 to 23, **characterized in that** the tool (86; 98; 120; 138; 176) is formed with a curved transversal cross section.

26. The apparatus in accordance with one of claims 1 to 23, **characterized in that** the tool (120) comprises a flute shaped or shovel shaped work portion (124).

27. The apparatus in accordance with one of claims 1 to 23 **characterized in that** the free end face (100; 108) of the tool (98; 106) comprises a portion being inclined with respect to the longitudinal axis of the tool or being curved in the sense of a flute.

28. The apparatus in accordance with claim 27, **characterized in that** the free end face (108) of the tool (106) comprises a portion extending perpendicular to the longitudinal axis of the tool.

29. The apparatus in accordance with one of claims 1 to 23, **characterized in that** the tool (134) comprises a shaft (136) and a transversal work portion (138) connected to the free end of the shaft.

30. The apparatus in accordance with one of claims 1 to 23, **characterized in that** the tool (148) comprises a chisel tip (154).

31. The apparatus in accordance with one of claims 1 to 23, **characterized in that** the tool (156) has a geometry corresponding to the geometry of a dental root channel (158).

32. The apparatus in accordance with one of claims 1 to 31, **characterized in that** the tool has an end portion vibrating in a direction being transversal to the longitudinal axis thereof.

33. The apparatus as in one of claims 1 to 32 **characterized by** at least two different shaping tools (80; 86) adapted to be sinked into the tissue in spaced parallel relationship and cooperating to define the geometry of a cavity to be made in the tissue.

## Revendications

1. Dispositif de préparation aux ultrasons de tissus durs ou mous d'animaux ou d'êtres humains, ainsi que de matériaux de remplacement de dents ou d'os avec un générateur de vibrations ultrasonores (16), ainsi qu'un outil (36) commandé par celui-ci, dans lequel est prévue entre le générateur de vibrations (16) et l'outil une tête de renvoi des ultrasons (28) qui présente un élément d'entrée (33) pouvant être entraîné par déplacement parallèlement à l'axe longitudinal (H) du générateur de vibrations (16) et un élément de sortie moteur (34), ce dernier étant mobile le long d'un axe (W) qui forme avec l'axe du générateur de vibrations un angle compris entre 60° et 120°, et dans lequel l'outil (36) est relié à l'élément de sortie moteur (34) de la tête de renvoi (28), la tête de renvoi (28) présentant un corps vibrant qui présente la forme d'une bague, d'un disque ou d'une boule creuse et qui présente une fréquence de résonance qui coïncide avec la fréquence de travail du générateur de vibrations (16), le diamètre du corps vibrant étant inférieur à 30 mm, de préférence inférieur à 20 mm.

2. Dispositif selon la revendication 1, **caractérisé en ce que** l'outil (36 ; 80 ; 86 ; 96 ; 98 ; 106 ; 120 ; 134 ; 140 ; 148 ; 156 ; 176) vibre parallèlement à son axe longitudinal.

3. Dispositif selon la revendication 2, **caractérisé en ce que** l'angle entre l'axe longitudinal (H) du générateur de vibrations (16) et l'axe longitudinal (W) de l'outil (36 ; 80 ; 86 ; 96 ; 98 ; 106 ; 120 ; 134 ; 140 ; 148 ; 156 ; 176) est de 90°.

4. Dispositif selon l'une des revendications 1 à 3, **caractérisé en ce que** l'ensemble du système qui est formé par le générateur de vibrations (16), la tête de renvoi (28) et l'outil (36 ; 80 ; 86 ; 96 ; 98 ; 106 ; 120 ; 134 ; 140 ; 148 ; 156 ; 176) vibre à la fréquence de résonance.

5. Dispositif selon l'une des revendications 1 à 4, **caractérisé en ce que** l'outil (80 ; 86 ; 156 ; 176) représente la forme d'un corps d'obturation moulé (88 ; 92 ; 176), qui est fabriqué à partir d'un matériau de remplacement de tissu, par exemple d'un matériau de remplacement de dents, et **en ce que** l'outil (80 ; 86 ; 156 ; 176) et la tête de renvoi (28) sont conçus de façon telle que l'outil (80 ; 86 ; 156 ; 176) peut être enclavé au moins partiellement dans le matériau à traiter par vibrations.

6. Dispositif selon la revendication 5, **caractérisé par** un jeu de différents outils (80 ; 86 ; 156 ; 176) qui sont conçus pour différentes tailles de dents et/ou différentes profondeurs minimum de préparations, qui sont par exemple spécifiques au matériau de restauration utilisé, et représentent des moules négatifs correspondant à différents corps d'obturation moulés (88 ; 92 ; 176).

7. Dispositif selon la revendication 6, **caractérisé par** un jeu de corps d'obturation dentaire (88; 92; 176) qui se présente sous les mêmes formes et échelonnements de taille que les outils (80 ; 86 ; 156 ; 176) utilisés pour la préparation cavitaire.

8. Dispositif selon la revendication 6 ou 7, **caractérisé en ce que** les corps d'obturation moulés (88 ; 92 ; 176) se composent d'un matériau céramique ou d'un matériau composite polymère ou d'un métal, par exemple du titane, et **en ce que** leurs surfaces sont au moins partiellement humidifiées avec des matériaux de fixation, en particulier des adhésifs polymères ou des ciments, ou reliées par engagement de forme ou par adhérence.

9. Dispositif selon l'une des revendications 1 à 8, **caractérisé en ce que** l'outil (140) comprend un élément de travail (144) qui se compose d'un matériau qui s'échauffe sous l'action des ultrasons.

10. Dispositif selon la revendication 9, **caractérisé en ce que** l'élément de travail (144) de l'outil (140) s'échauffe par chaleur de friction, en particulier par chaleur de friction interne, l'élément de travail se composant à cet égard de préférence de plusieurs couches de matériaux différentes.

11. Dispositif selon l'une des revendications 1 à 10, **caractérisé par** au moins un équipement (46, 50, 52 ; 164, 166, 168) permettant d'amener un milieu de traitement abrasif (48 ; 70) vers l'outil (36 ; 80 ; 86 ; 98 ; 106 ; 120 ; 134 ; 148 ; 156 ; 176), le milieu de traitement abrasif (48 ; 170) contenant de préférence de petites particules abrasives de bourrage (60) présentant un diamètre moyen de particules inférieur à 5 µm.

12. Dispositif selon la revendication 11, **caractérisé en ce que** les particules abrasives de bourrage (60) présentent une taille moyenne de particules environ égale à 1 µm.

13. Dispositif selon la revendication 11 ou 12, **caractérisé en ce que** l'on compte parmi les particules abrasives des particules de bourrage (60) qui se composent de matériaux formateurs d'oxydes et/ou de silicates et/ou de carbures et/ou de nitrures et/ou contenant des polymères, et **en ce que** la surface des particules de bourrage peut au moins partiellement réaliser une liaison chimique au moins partielle avec des silanes et/ou des polymères, et/ou, au moins partiellement sous l'action de matériaux d'obturation pour joints au moins partiellement gélifiants contenant des acides, en particulier des ciments au verre ionomère ou des compomères durcissant sous l'action de la lumière ou sous une action chimique, s'intégrer dans un gel qui se forme, une fraction en volume de particules de bourrage allant jusqu'à 2 à 20, de préférence environ 10 fractions en volume de particules abrasives étant de préférence utilisées.

14. Dispositif selon l'une des revendications 11 à 13, **caractérisé en ce que** le milieu de traitement (130) a la forme d'au moins un gel.

15. Dispositif selon la revendication 14, **caractérisé en ce que** le milieu de traitement (130) présente une viscosité modifiée approximativement par l'ajout d'un aérosil ou de substances de support gélifiantes, par exemple de la gélatine, et **en ce que** le milieu de traitement en gel est au moins partiellement fluidifié par l'action des ultrasons.

16. Dispositif selon la revendication 14 ou 15, **caractérisé en ce que** la composante de base du milieu de traitement (130) est un gel de glycérine ou un gel de chlorhexidine à environ 1 à 10 %, ou une gélatine.

17. Dispositif selon l'une des revendications 5 à 16, **caractérisé en ce que** l'outil et un corps d'obturation moulé à utiliser dans une cavité tissulaire sont formés conjointement par un corps d'obturation moulé (156 ; 176) fabriqué à partir d'un matériau dur compatible avec les tissus.

18. Dispositif selon la revendication 17, **caractérisé en ce que** le corps d'obturation moulé (156 ; 176) se compose de matériaux de céramique oxydée ou de céramique non oxydée ou d'un matériau métallique, par exemple du titane.

19. Dispositif selon la revendication 17 ou 18, **caractérisé en ce que** le corps d'obturation moulé (176) présente un élément de fixation (178) pouvant être relié à l'élément de sortie entraînant (34; 162d) de la tête de renvoi (28), qui est relié, par exemple vissé, coincé ou soudé, par un point destiné à la rupture (180) au segment du corps d'obturation moulé (176) à insérer dans la cavité tissulaire.

20. Dispositif selon l'une des revendications 11 à 19, **caractérisé en ce que** le milieu de traitement (48 ; 170) contient des particules abrasives dont le diamètre correspond de préférence approximativement à la course totale de la vibration ultrasonore de l'outil (36 ; 80 ; 86 ; 98 ; 106 ; 120 ; 134 ; 148 ; 156 ; 176), une fraction en volume de particules abrasives de préférence de 3 à 50, de préférence aussi de 5 à 20, et de préférence encore d'environ 10 fractions en volume de fluide, étant utilisée.

21. Dispositif selon l'une des revendications 11 à 20, **caractérisé en ce que** les particules abrasives (56, 58, 60) présentent une surface hydrophile ou un revêtement de surface hydrophile.

22. Dispositif selon l'une des revendications 11 à 21, **caractérisé par** un autre équipement (46, 50, 52) permettant d'amener le milieu de traitement (48) vers le côté externe de l'outil (36 ; 80 ; 86 ; 98 ; 106 ; 120 ; 134 ; 148 ; 156 ; 176).

23. Dispositif selon l'une des revendications 1 à 22, **caractérisé en ce que** l'outil (36 ; 80 ; 86 ; 96 ; 106 ; 120 ; 134 ; 148 ; 156) est au moins partiellement creux et est relié à un équipement (164, 166, 168) permettant d'amener le milieu de traitement abrasif (170).

24. Dispositif selon l'une des revendications 1 à 23, **caractérisé en ce que** l'outil (96) présente au moins une tête de vibrateur bombée.

25. Dispositif selon l'une des revendications 1 à 23, **caractérisé en ce que** l'outil (86 ; 98 ; 120 ; 148 ; 176) présente une section transversale arquée.

26. Dispositif selon l'une des revendications 1 à 23, **caractérisé en ce que** l'outil (120) présente un élément de travail (124) en forme de rigole ou de pelle.

27. Dispositif selon l'une des revendications 1 à 23, **caractérisé en ce que** l'extrémité frontale libre (100 ; 108) de l'outil (98 ; 106) présente un segment s'étendant en oblique vers l'axe longitudinal de l'outil ou arqué au sens d'un congé creux.

28. Dispositif selon la revendication 27, **caractérisé en ce que** l'extrémité frontale libre (108) de l'outil (106) présente un segment s'étendant perpendiculairement à l'axe longitudinal de l'outil.

29. Dispositif selon l'une des revendications 1 à 23, **caractérisé en ce que** l'outil (134) présente un manche (136) et un élément de travail transversal (138) fixé à son extrémité libre.

30. Dispositif selon l'une des revendications 1 à 23, **caractérisé en ce que** l'outil (148) présente une pointe de burin (154).

31. Dispositif selon l'une des revendications 1 à 23, **caractérisé en ce que** l'outil (156) possède une géométrie qui correspond à la géométrie d'un canal radiculaire (158).

32. Dispositif selon l'une des revendications 1 à 31, **caractérisé en ce que** l'outil présente un segment d'extrémité vibrant transversalement par rapport à son axe longitudinal.

33. Dispositif selon l'une des revendications 1 à 33, **caractérisé par** au moins deux outils de moulage (86 ; 96) différents pouvant être enclavés parallèlement et de façon décalée dans le tissu, et qui délimitent ensemble la forme d'une cavité à générer dans le tissu.
